# EUROPEAN PATENT SPECIFICATION

(11) **EP 1 843 796 B1**
(45) Date of publication and mention of the grant of the patent: **27.02.2013**
(21) Application number: 06719079.3
(22) Date of filing: 23.01.2006
(51) Int. Cl.: A61L 15/60, A61L 15/26, B32B 5/14, B32B 5/16, C08G 18/08, C08G 18/10, C08G 18/48, C09D 175/04

(54) **ABSORBENT STRUCTURE WITH IMPROVED WATER-ABSORBING MATERIAL**
ABSORPTIONSSTRUKTUR MIT VERBESSERTEM WASSERABSORBIERENDEM MATERIAL
STRUCTURE ABSORBANTE COMPRENANT UNE MATIERE ABSORBANT L'EAU

(30) Priority: 04.02.2005 US 650344 P
(43) Date of publication of application: 17.10.2007
(73) Proprietor: The Procter & Gamble Company, Cincinnati, OH 45202 (US)
(72) Inventor: SCHMIDT, Mattias, 65510 Idstein (DE); MEYER, Axel, 60486 Frankfurt (DE); FOSSUM, Renae, Dianna, Middletown, Ohio 45044 (US); RIEGEL, Ulrich, 66849 Landstuhl (DE); DANIEL, Thomas, Dr., 67165 Waldsee (DE); BRUHNS, Stefan, Dr., 86165 Mannheim (DE); ELLIOT, Mark, Bsc, 67063 Ludwigshafen (DE); MADSEN, James Scott, Cottage Grove, Wisconsin 53527 (US)
(74) Representative: Heide, Ute
(86) International application number: PCT/US2006/002111
(87) International publication number: WO 2006/083582

(56) References cited:
- EP-A- 0 612 533
- EP-A- 0 900 571
- EP-A- 1 013 291
- WO-A-99/47072
- WO-A-2005/014065
- WO-A-2006/042704
- US-A- 5 731 365
- US-A- 5 851 672

## Description

### FIELD OF THE INVENTION

This invention relates to improved absorbent structures containing improved water-absorbing material having a specific coating of elastomeric, film-forming polymers and/or which are made by a specific coating process.

The invention also relates to diapers, adult incontinence articles and catamenial devices, such as sanitary napkins, comprising said absorbent structure of the invention.

### BACKGROUND TO THE INVENTION

An important component of disposable absorbent articles such as diapers is an absorbent core structure comprising water-absorbing polymers, typically hydrogel-forming water-absorbing polymers, also referred to as absorbent gelling material, AGM, or super-absorbent polymers, or SAP's. This polymer material ensures that large amounts of bodily fluids, e.g., urine, can be absorbed by the article during its use and locked away, thus providing low rewet and good skin dryness.

Especially useful water-absorbing polymers or SAP's are often made by initially polymerizing unsaturated carboxylic acids or derivatives thereof, such as acrylic acid, alkali metal (e.g., sodium and/or potassium) or ammonium salts of acrylic acid, alkyl acrylates, and the like in the presence of relatively small amounts of di- or poly-functional monomers such as N,N'-methylenebisacrylamide, trimethylolpropane triacrylate, ethylene glycol di(meth)acrylate, or triallylamine. The di- or poly-functional monomer materials serve to lightly cross-link the polymer chains thereby rendering them water-insoluble, yet water-absorbing. These lightly crosslinked absorbent polymers contain a multiplicity of carboxylate groups attached to the polymer backbone. It is generally believed that the neutralized carboxylate groups generate an osmotic driving force for the absorption of body fluids by the crosslinked polymer network. In addition, the polymer particles are often treated as to form a surface cross-linked layer on the outer surface in order to improve their properties in particular for application in baby diapers.

Water-absorbing (hydrogel-forming) polymers useful as absorbents in absorbent members and articles such as disposable diapers need to have adequately high absorption capacity, as well as adequately high gel strength. Absorption capacity needs to be sufficiently high to enable the absorbent polymer to absorb significant amounts of the aqueous body fluids encountered during use of the absorbent article. Together with other properties of the gel, gel strength relates to the tendency of the swollen polymer particles to resist deformation under an applied stress. The gel strength needs to be high enough in the absorbent member or article so that the particles do not deform and fill the capillary void spaces to an unacceptable degree causing so-called gel blocking. This gel-blocking inhibits the rate of fluid uptake or the fluid distribution, i.e., once gel-blocking occurs, it can substantially impede the distribution of fluids to relatively dry zones or regions in the absorbent article and leakage from the absorbent article can take place well before the water-absorbing polymer particles are fully saturated or before the fluid can diffuse or wick past the "gel blocking" particles into the rest of the absorbent article. Thus, it is important that the water-absorbing polymers (when incorporated in an absorbent structure or article) maintain a high wet-porosity and have a high resistance against deformation thus yielding high permeability for fluid transport through the swollen gel bed.

Absorbent polymers with relatively high permeability can be made by increasing the level of internal crosslinking or surface crosslinking, which increases the resistance of the swollen gel against deformation by an external pressure such as the pressure caused by the wearer, but this typically also reduces the absorbent capacity of the gel which is undesirable. It is a significant draw back of this conventional approach that the absorbent capacity has to be sacrificed in order to gain permeability. The lower absorbent capacity must be compensated by a higher dosage of the absorbent polymer in hygiene articles which, for example,, leads to difficulties with the core integrity of a diaper during wear. Hence, special, technically challenging and expensive fixation technologies are required to overcome this issue in addition to the higher costs that are incurred because of the required higher absorbent polymer dosing level.

Because of the trade-off between absorbent capacity and permeability in the conventional approach, it is extremely difficult to produce absorbent polymers that show improved properties regarding absorbent capacity and permeability versus what is described by the following empirical equation: $Log ⁢ \left(CS-SFCʹ/150\right) \leq 3.36 - 0.133 \times CS - CRC$ and it is even more difficult to produce absorbent polymers that show improved properties regarding absorbent capacity and permeability versus what is described by the following empirical equation: $Log ⁢ \left(CS-SFCʹ/150\right) \leq 2.5 - 0.095 \times CS - CRC$
It is therefore very desirable to produce absorbent polymers that fulfil the following equations (3) or (4) or preferred (3) and (4): $Log ⁢ \left(CS-SFCʹ/150\right) > 3.36 - 0.133 \times CS - CRC$ $Log ⁢ \left(CS-SFCʹ/150\right) > 2.5 - 0.095 \times CS - CRC$
In all equations above, CS-SFC' = CS-SFC × 10⁷ and the dimension of 150 is [cm³s/g].

Often the surface crosslinked water-absorbing polymer particles are constrained by the surface-crosslinked shell and cannot absorb and swell sufficiently, and/or the surface-crosslinked shell is not strong enough to withstand the stresses of swelling or the stresses associated with performance under load.

As a result thereof the coatings or shells of the water-absorbing polymers, as used in the art, including surface cross-linking 'coatings', break when the polymer swells significantly or that the 'coatings' break after having been in a swollen state for a period of time. Often the coated and/or surface-crosslinked water-absorbing polymers or super-absorbent material known in the art deform significantly in use thus leading to relatively low porosity and permeability of the gel bed in the wet state.

The present invention thus has for its objective to provide absorbent structures containing a water-absorbing material with water-absorbing polymers with a more advantageous modification of the surface whose integrity is preserved during the swelling and preferably also during the lifetime of the hygiene article manufactured using this absorbent polymer.

EP-A-0 703 265 teaches the treatment of hydrogel with film-forming polymers such as acrylic/methacrylic acid dispersions to produce abrasion-resistant absorbents. The treating agents identified include polyurethanes. However, the absorbent particles obtained therein give unsatisfactory absorption values, especially with regard to CS-CRC and CS-SFC. More particularly, the reference cited does not teach how to produce uniform coatings that retain their mechanical properties to a sufficient degree during swelling and during use.

The objective of this invention accordingly is to provide absorbent structures comprising water-absorbing material having high core shell centrifuge retention capacity (CS-CRC), and high core shell saline flow conductivity (CS-SFC), and typically high core shell absorbency under load (CS-AUL).

We have found that this objective is achieved by absorbent structures that comprise water-absorbing material comprising water-absorbing polymer particles with specific elastomeric film-forming polymer coatings, i.e., polyetherpolyurethane coatings, or specific spray coated heat-treated coatings,and/or that this is achieved by absorbent structures that comprise water-absorbing material obtainable by a process comprising the steps of :
a) spray-coating water-absorbing polymeric particles with an elastic film-forming polymer in a fluidized bed reactor in the range from 0°C to 50°C and
b) heat-treating the coated particles at a temperature above 50°C.

### SUMMARY OF THE INVENTION

The invention provides, in a first embodiment, an absorbent structure for use in an absorbent article, said absorbent structure comprising a water-absorbing material, which comprises water-absorbing polymer particles coated with polyether polyurethane that has polyalkylene oxide units in the also side chains.

The invention provides an absorbent structure for use in an absorbent article, said absorbent structure comprising a water-absorbing material obtainable by a process comprising the steps of:
a) spray-coating water-absorbing polymer particles with said polyurethane, elastomeric polymers at temperatures in the range from 0°C to 50°C, to obtain coated particles; and
b) heat-treating the coated particles at a temperature above 50°C.

The invention also provides an absorbent structure for use in an absorbent article, said absorbent structure comprising a water-absorbing material, which comprises coated water-absorbing polymer particles that have a heat-treated, spray-coating of said polyurethane elastomeric polymers, typically as obtained by the process above, typically having a coating level of less than 10% or preferably less than 5% by weight (of the water-absorbing polymers), as described herein.

Preferably, the coating comprises at least one polyetherpolyurethane that has a fraction of alkylene glycol units in the side chains from 10% to 90% by weight based on the total weight of the polyetherpolyurethane.

Preferably, the polyetherpolyurethane has ethylene oxide units in its side chains, and optionally in its main chain(s), whereby the fraction of ethylene oxide units in the side chains of the polyetherpolyurethane is not less than 12% by weight and the fraction of ethylene oxide units in the main chains of the polyetherpolyurethane is not more than 30% by weight based on the total weight of the polyetherpolyurethane.

The absorbent structure is preferably an absorbent article or part of or incorporated in an absorbent article, such as a diaper, an adult incontinence product, or a catamenial device, such as a sanitary napkin. For example, it may be the storage layer of such an article, and it then preferably has a density of at least about 0.4 g/cm³,and/or it then preferably comprises less than 40% or even more preferably less than 30%, or even more preferably less than 20% by weight (of the water-swellable material) of absorbent fibrous material, and it may even be preferred that it comprises less than 10% by weight of fibrous absorbent material or even no fibrous absorbent material at all.

### BRIEF DESCRIPTION OF THE DRAWINGS

Fig. 1 is a schematic view of the permeability equipment setup.
Fig. 2 is a detailed view of the SFC cylinder/plunger apparatus.
Fig. 3 is a view of the SFC plunger details.

### DETAILED DESCRIPTION

### Absorbent structures

"Absorbent structure" refers to any three dimensional structure, comprising water-absorbing material, useful to absorb and retain liquids, such as urine, menses or blood.

"Absorbent article" refers to devices that absorb and retain liquids (such as blood, menses and urine), and more specifically, refers to devices that are placed against or in proximity to the body of the wearer to absorb and contain the various exudates discharged from the body. Absorbent articles include but are not limited to diapers, including training pants, adult incontinence briefs, diaper holders and liners, sanitary napkins and the like.

"Diaper" refers to an absorbent article generally worn by infants and incontinent persons about the lower torso.

"Disposable" is used herein to describe articles that are generally not intended to be laundered or otherwise restored or reused (i.e., they are intended to be discarded after a single use and, preferably, to be recycled, composted or otherwise disposed of in an environmentally compatible manner).

The absorbent structure typically comprises the water-absorbing material herein and a structuring material, such as a core wrap or wrapping material, support layer for the water-absorbing material or structuring agent such as described below.

The absorbent structure is typically, or forms typically part of, an absorbent article, and preferably disposable absorbent articles, such as preferably sanitary napkins, panty liners, and more preferably adult incontinence products, diapers, and training pants.

If the absorbent structure is part of a disposable absorbent article, then the absorbent structure of the invention is typically that part of an absorbent article which serves to store and/or acquire bodily fluids, the absorbent structure may be the storage layer of an absorbent article, or the acquisition layer, or both, either as two or more layers or as unitary structure.

The absorbent structure may be a structure that consists of the water-absorbing material and that is then shaped into the required three-dimensional structure, or preferably, it may comprise additional components, such as those used in the art for absorbent structures.

Preferred is that the absorbent structure also comprise one or more support or wrapping materials, such as foams, films, woven webs and/or nonwoven webs, as known in the art, such as spunbond, meltblown and/or carded nonwovens. One preferred material is a so-called SMS material, comprising a spunbonded, a melt-blown and a further spunbonded layer. Highly preferred are permanently hydrophilic nonwovens, and in particular nonwovens with durably hydrophilic coatings. An alternative preferred material comprises a SMMS-structure. The top layer and the bottom layer may be provided from two or more separate sheets of materials or they may be alternatively provided from a unitary sheet of material.

Preferred non-woven materials are provided from synthetic fibers, such as PE, PET and most preferably PP. As the polymers used for nonwoven production are inherently hydrophobic, they are preferably coated with hydrophilic coatings, e.g., coated with nanoparticles, as known in the art.

Preferred nonwoven materials and absorbent structures using such materials are described in, for example,, co-pending applications US2004/03625, US2004/03624, and US2004/03623 and in US 2004/0162536, EP1403419-A, WO2002/0192366, EP 1470281-A and EP1470282-A.

The absorbent structure may also comprise a structuring agent or matrix agent, such as absorbent fibrous material, such as airfelt fibers, and/or adhesive, which each may serve to immobilize the water-absorbing material.

Because the water-absorbing material herein has an excellent permeability, even when swollen, there is no need for large amounts of structuring agents, such as absorbent fibrous material (airfelt), as normally used in the art.

Thus, preferably a relatively low amount or no absorbent fibrous (cellulose) material is used in the absorbent structure. Thus, it may be preferred that said structure herein comprises large amounts of the water-absorbing material herein and only very little or no absorbent (cellulose) fibers, preferably less than 20% by weight of the water-absorbing material, or even less than 10% by weight of the water-absorbing material, or even less than 5% by weight.

Preferred absorbent structures herein comprise a layer of a substrate material such as the core-wrap materials described herein, and thereon a water-absorbing material layer, optionally as a discontinuous layer, and thereon a layer of an adhesive or thermoplastic material or preferably a (fibrous) thermoplastic adhesive material, which is laid down onto the layer of water-absorbing material. Preferred may be that the thermoplastic or adhesive layer is then in direct contact with the water-absorbing material, but also partially in direct contact with the substrate layer, where the substrate layer is not covered by the absorbent polymeric material. This imparts an essentially three-dimensional structure to the (fibrous) layer of thermoplastic or adhesive material, which in itself is essentially a two-dimensional structure of relatively small thickness (in z-direction), as compared to the extension in x- and y-direction.

Thereby, the thermoplastic or adhesive material provides cavities to hold the water-absorbing material and thereby immobilizes this material. In a further aspect, the thermoplastic or adhesive material bonds to the substrate and thus affixes the water-absorbing material to the substrate.

In this embodiment, it may be preferred that no absorbent fibrous material is present in the absorbent structure.

The thermoplastic composition may comprise, in its entirety, a single thermoplastic polymer or a blend of thermoplastic polymers, having a softening point, as determined by the ASTM Method D-36-95 "Ring and Ball", in the range between 50°C and 300°C, or alternatively the thermoplastic composition may be a hot melt adhesive comprising at least one thermoplastic polymer in combination with other thermoplastic diluents such as tackifying resins, plasticizers and additives such as antioxidants.

The thermoplastic polymer has typically a molecular weight (Mw) of more than 10,000 and a glass transition temperature (Tg) usually below room temperature. A wide variety of thermoplastic polymers are suitable for use in the present invention. Such thermoplastic polymers are preferably water insensitive. Exemplary polymers are (styrenic) block copolymers including A-B-A triblock structures, A-B diblock structures and (A-B)n radial block copolymer structures wherein the A blocks are non-elastomeric polymer blocks, typically comprising polystyrene, and the B blocks are unsaturated conjugated diene or (partly) hydrogenated versions of such. The B block is typically isoprene, butadiene, ethylene/butylene (hydrogenated butadiene), ethylene/propylene (hydrogenated isoprene), and mixtures thereof.

Other suitable thermoplastic polymers that may be employed are metallocene polyolefins, which are ethylene polymers prepared using single-site or metallocene catalysts. Therein, at least one comonomer can be polymerized with ethylene to make a copolymer, terpolymer or higher order polymer. Also applicable are amorphous polyolefins or amorphous polyalphaolefins (APAO) which are homopolymers, copolymers or terpolymers of C2 to C8 alphaolefins.

The resin has typically a Mw below 5,000 and a Tg usually above room temperature, typical concentrations of the resin in a hot melt are in the range of 30 - 60%. The plasticizer has a low Mw of typically less than 1,000 and a Tg below room temperature, a typical concentration is 0 -15%.

Preferably the adhesive is present in the forms of fibres throughout the core, i.e., the adhesive is fiberized or fibrous.

Preferably, the fibres will preferably have an average thickness of 1 - 50 micrometer and an average length of 5 mm to 50 cm.

Preferably, the absorbent structure, in particular when no or little absorbent fibres are present, as described above, has a density greater than about 0.4 g/cm³. Preferably, the density is greater than about 0.5 g/cm³, more preferably greater than about 0.6 g/cm³.

Preferred absorbent structures can, for example, be made as follows:
a) providing a substrate material that can serve as a wrapping material;
b) depositing the water-absorbing material herein onto a first surface of the substrate material, preferably in a pattern comprising at least one zone which is substantially free of water-absorbing material, and the pattern comprising at least one zone comprising water-absorbing material, preferably such that openings are formed between the separate zones with water-absorbing material;
c) depositing a thermoplastic material onto the first surface of the substrate material and the water-absorbing material, such that portions of the thermoplastic material are in direct contact with the first surface of the substrate and portions of the thermoplastic material are in direct contact with the water-absorbing material; and
d) then typically closing the above by folding the substrate material over, or by placing another substrate matter over the above.

The absorbent structure may comprise an acquisition layer and a storage layer, which may have the same dimensions, however it may be preferred that the acquisition layer is laterally centered on the storage layer with the same lateral width but a shorter longitudinal length than storage layer. The acquisition layer may also be narrower than the storage layer while remaining centered thereon. Said another way, the acquisition layer suitably has an area ratio with respect to storage layer of 1.0, but the area ratio may preferably be less than 1.0, e.g., less than about 0.75, or more preferably less than about 0.50.

For absorbent structures and absorbent articles designed for absorption of urine, it may be preferred that the acquisition layer is longitudinally shorter than the storage layer and positioned such that more than 50% of its longitudinal length is forward of transverse axis of the absorbent structure or of the absorbent article herein. This positioning is desirable so as to place acquisition layer under the point where urine is most likely to first contact absorbent structure or absorbent article.

Also, the absorbent core, or the acquisition layer and/or storage layer thereof, may comprise an uneven distribution of water-absorbing material basis weight in one or both of the machine and cross directions. Such uneven basis weight distribution may be advantageously applied in order to provide extra, predetermined, localized absorbent capacity to the absorbent structure or absorbent article.

The absorbent structure of the invention may be, or may be part of an absorbent article, typically it may be the absorbent core of an absorbent article, or the storage layer and/or acquisition layer of such an article.

Preferred (disposable) absorbent article comprising the absorbent structure of the invention are sanitary napkins, panty liners, adult incontinence products and infant diapers or training or pull-on pants, whereby articles which serve to absorb urine, e.g., adult incontinence products, diapers and training or pull-on pants are the most preferred articles herein.

Preferred articles herein have a topsheet and a backsheet, which each have a front region, back region and crotch region, positioned therein between. The absorbent structure of the invention is typically positioned in between the topsheet and backsheet. Preferred backsheets are vapor pervious but liquid impervious. Preferred topsheet materials are at least partially hydrophilic; preferred are also so-called apertured topsheets. Preferred may be that the topsheet comprises a skin care composition, e.g., a lotion.

These preferred absorbent articles typically comprise a liquid impervious (but preferably air or water vapour pervious) backsheet, a fluid pervious topsheet joined to, or otherwise associated with the backsheet. Such articles are well known in the art and fully disclosed in various documents mentioned throughout the description.

Because the water-absorbing material herein has a very high absorbency capacity, it is possible to use only low levels of this material in the absorbent articles herein. Preferred are thus thin absorbent articles, such as adult and infant diapers, training pants, sanitary napkins comprising an absorbent structure of the invention, the articles having an average caliper (thickness) in the crotch region of less than 1.0 cm, preferably less than 0.7cm, more preferably less than 0.5cm, or even less than 0.3cm (for this purpose alone, the crotch region being defined as the central zone of the product, when laid out flat and stretched, having a dimension of 20% of the length of the article and 50% of the width of the article).

Because the water-absorbing material herein have a very good permeability, there is no need to have large amounts of traditional structuring agents presents, such as absorbent fibres, such as airfelt, and the may thus be omitted or only used in very small quantities, as described above. This further helps to reduce the thickness of the absorbent structure, or absorbent articles herein.

Preferred articles according to the present invention achieve a relatively narrow crotch width, which increases the wearing comfort. A preferred article according to the present invention achieves a crotch width of less than 100 mm, 90 mm, 80 mm, 70 mm, 60 mm or even less than 50 mm, as measured along a transversal line with is positioned at equal distance to the front edge and the rear edge of the article, or at the point with the narrowest width. Hence, preferably an absorbent structure according to the present invention has a crotch width as measured along a transversal line with is positioned at equal distance to the front edge and the rear edge of the core which is of less than 100 mm, 90 mm, 80 mm, 70 mm, 60 mm or even less than 50 mm. It has been found that for most absorbent articles the liquid discharge occurs predominately in the front half.

A preferred diaper herein has a front waist band and a back waist band, whereby the front waist band and back waist band each have a first end portion and a second end portion and a middle portion located between the end portions, and whereby preferably the end portions comprise each a fastening system, to fasten the front waist band to the rear waist band or whereby preferably the end portions are connected to one another, and whereby the middle portion of the back waist band and/or the back region of the backsheet and/or the crotch region of the backsheet comprises a landing member, preferably the landing member comprising second engaging elements selected from loops, hooks, slots, slits, buttons, magnets. Most preferred are hooks, adhesive or cohesive second engaging elements. Preferred may be that the engaging elements on the article, or preferably diaper are provided with a means to ensure they are only engage able at certain moments, for example, they may be covered by a removable tab, which is removed when the engaging elements are to be engaged and may be re-closed when engagement is no longer needed, as described above.

Preferred diapers and training pants herein have one or more sets of leg elastics and/or barrier leg cuffs, as known in the art.

Preferred may also be that the topsheet has an opening, preferably with elastication means along the length thereof, where through waste material can pass into a void space above the absorbent structure, and which ensures it is isolated in this void space, away from the wearer's skin.

### Water-absorbing material

The water-absorbing material herein is such that it swells in water by absorbing the water; it may thereby form a gel. It may also absorb other liquids and swell. Thus, when used herein, 'water-absorbing' means that the material absorbs water, and typically swells in water, but typically also (in) other liquids or solutions, preferably water based liquids such as 0.9% saline and urine.

The water-absorbing material is solid; this includes gels, and particles, such as flakes, fibers, agglomerates, large blocks, granules, spheres, and other forms known in the art as 'solid' or 'particles'.

The coated water-absorbing polymers may be present in the water-absorbing material mixed with other components, such as fibers, (fibrous) glues, organic or inorganic filler materials or flowing aids, process aids, anti-caking agents, odor control agents, coloring agents, coatings to impart wet stickiness, hydrophilic surface coatings, etc.

The water-absorbing material herein preferably comprises less than 20% by weight of water, or even less than 10% or even less than 8% or even less than 5%, or even no water. The water content of the water-absorbing material can be determined by the Edana test, number ERT 430.1-99 (February 1999) which involves drying the water-absorbing material at 105°Celsius for 3 hours and determining the moisture content by the weight loss of the water-absorbing materials after drying.

The water-absorbing material herein comprises coated water-absorbing polymer particles, said particles preferably being present at a level of at least 50% to 100% by weight (of the water-absorbing material) or even from 80% to 100% by weight, and most preferably the material consists of said water-absorbing particles. Said water-absorbing particles of the water-absorbing material preferably have a core-shell structure, as described herein, whereby the core preferably comprises said water-absorbing polymer(s), which are typically also particulate.

The water-absorbing material herein has a very high permeability or porosity, as represented by the CS-SFC value, as measured by the method set out herein.

The CS-SFC of the water-absorbing material herein is typically at least 10 x 10⁻⁷cm³sec/g, but preferably at least 30 x 10⁻⁷cm³sec/g or more preferably at least 50 10⁻⁷cm³sec/g or even more preferably at least 100 10⁻⁷cm³sec/g. It may even be preferred that the CS-SFC is at least 500 10⁻⁷cm³sec/g or even more preferably at least 1000 10⁻⁷cm³sec/g, and it has been found to be even possible to have a CS-SFC of 2000 10⁻⁷cm³sec/g or more.

Typically, the water-absorbing material is particulate, having preferably particle sizes and distributions, which are about equal to the preferred particle sizes/distributions of the water-absorbing polymer particles, as described herein below, even when these particles comprise a shell of, for example,, elastomeric polymers, because this shell is typically very thin and does not significantly impact the particle size of the particles of the water-absorbing material.

It is possible that the water-absorbing material comprises two or more layers of coating agent (shells), obtainable by coating the water-absorbing polymers twice or more. This may be the same coating agent or a different coating agent. However, preference for economic reasons is given to a single coating with a film-forming polymer and preferably with a polyurethane.

Preference is given to a water-absorbing material whose Core Shell Centrifuge Retention Capacity (CS-CRC) value is not less than 20 g/g, preferably not less than 25 g/g.

Surprisingly it has been found that, in contrast to water-absorbing polymer particles known in the art, the particles of the water-absorbing material herein are typically substantially spherical when swollen, for example, when swollen by the method set out in the 4 hour CCRC test, described below. Namely, the particles are, even when swollen, able to withstand the average external pressure to such a degree that hardly any deformation of the particles takes place, ensuring the highly improved permeability.

The sphericity of the swollen particles can be determined (visualized) by, for example, the PartAn method or preferably by microscopy.

The water-absorbing material herein has a Saline Absorbent Capacity (SAC), a Saline Absorbent Capacity after grinding (SAC") and a QUICS value calculated therefrom, as defined by the methods described hereinafter. The difference between SAC" and SAC and thus the QUICS calculated therefrom can be used as a measure for the internal pressure exerted onto the core of the particles (containing water-absorbing polymer) of the water-absorbing material.

Highly preferred are water-absorbing materials with a QUICS of at least 15, or more preferably at least 20, or even more preferably at least 30, and preferably up to 200 or even more preferably up to 150 or even more preferably up to 100.

In particular, the water-absorbing materials herein have a particularly beneficial absorbency-distribution-index (ADI) of more than 1, preferably at least 2, more preferably at least 3, even more preferably at least 6 and most preferable of at least about 10, whereby the ADI is defined as: $ADI = \left(CS-SFC/\left(150*{10}^{- 7}⁢{cm}^{3}⁢sec/g\right)\right) / {10}^{2.5 - 0.095 \times \left(CS-CCRC/g/g\right)}$
CS-CCRC is the Cylinder Centrifuge Retention Capacity after 4 hours of swelling as set out in the test method section below.

Typically, the water-absorbing materials will have an ADI of not more than about 200 and preferably not more than 50.

### Coatings and preferred elastomeric film-forming polymers thereof

The water-absorbing material herein comprises water-absorbing particles, with a core-shell structure, whereby said core comprises water-absorbing polymer(s) and said shell (coating on said core) comprises elastomeric film-forming polymers, herein referred to as elastomeric polymers. Film forming means typically that the respective polymer can readily be made into a layer or coating, e.g., upon evaporation of the solvent in which it is dissolved or dispersed.

It should be understood that the coating or shell will be present on at least a portion of the surface of the core, referred to herein; this includes the embodiment that said coating or shell may form the outer surface of the particles, and the embodiment that the coating or shell does not form the outer surface of the particles.

In a preferred execution, the water-absorbing material comprises, or consists of, water-absorbing particles, which have a core formed by particulate water-absorbing polymer(s), as described herein, and this core forms the centre of the particles of the water-absorbing material herein, and the water-absorbing particles comprise each a coating or shell, which is present on substantially the whole outer surface area of said core.

In one preferred embodiment herein, the coating or shell is an essentially continuous coating layer around the water-absorbing polymer core, and said layer covers the entire surface of the polymer core, i.e., no regions of the core surface are exposed. Hereto, the coating or shell is typically formed by the preferred processes described herein after.

The coating or shell, preferably formed in the preferred process described herein, is preferably pathwise connected and more preferably, the shell is pathwise connected and encapsulating (completely circumscribing) the core, e.g., of water-absorbing polymer(s) (see, for example, E. W. Weinstein et. al., Mathworld - A Wolfram Web Resource for 'encapsulation' and 'pathwise connected'). The coating or shell is preferably a pathwise connected complete surface on the surface of the core. This complete surface consists of first areas where the shell is present and which are pathwise connected, e.g., like a network, but it may comprise second areas, where no shell is present, being, for example, micro pores, whereby said second areas are a disjoint union. Preferably, each second area, e.g., micropore, has a surface area of less than 0.1 mm², or even less than 0.01 mm² preferably less than 8000 µm², more preferably less than 2000 µm² and even more preferably less than 80µm². However, it is most preferred that no second areas are present, and that the shell forms a complete encapsulation around the core, e.g., of water-absorbing polymer(s).

As said above, the coating or shell comprises elastomeric film-forming polymers, preferably polyetherpolyurethanes, as described hereinafter. The coating is preferably applied by the method described hereinafter, e.g., preferably a dispersion or solution of the elastomeric film-forming polymers is sprayed onto the water-absorbing polymer particles by the preferred processes described herein. It has surprisingly been found that these preferred process conditions further improve the resistance of the shell against pressure, improving the permeability of the water-absorbing material whilst ensuring a good absorbency.

In one embodiment, the polymer has a tensile stress at break in the wet state of at least 1 MPa, or even at least 3 MPa and more preferably at least 5 MPa, or even at least 8 MPa. Most preferred materials have tensile stress at break of at least 10 MPa, preferably at least 40 MPa. This can be determined by the test method, described below.

In one embodiment, particularly preferred polymers herein are materials that have a wet secant elastic modulus at 400% elongation (SM_{wet} 400%) of at least 0.25 MPa, preferably at least about 0.50 MPa, more preferably at least about 0.75 or even at least 2.0 MPa, and most preferably of at least about 3.0 MPa as determined by the test method below.

The shell or coating herein, or the elastomeric polymers thereof, has in general a high shell tension, which is defined as the (Theoretical equivalent shell caliper) x (Average wet secant elastic modulus at 400% elongation), of 5 to 200 N/m, or preferably of 10 to 170N/m, or more preferably 20 to 130 N/m. In some embodiments it may be preferred to have a shell with a shell tension of 40N/m to 110N/m.

In one embodiment herein, where the water-absorbing polymers herein have been (surface) post-crosslinked (either prior to application of the coating described herein, or at the same time as applying said coating), it may even be more preferred that the shell tension is in the range from 15 N/m to 60N/m, or even more preferably from 20 N/m to 60N/m, or preferably from 40 to 60 N/m.

In yet another embodiment wherein the water-absorbing polymers are not surface-crosslinked, it may even be more preferred that said shell tension is in the range from more than 60 N/m to 110 N/m.

The coating is preferably at least moderately water-permeable (breathable) with a moisture vapor transmission rate (MVTR; as can be determined by the method set out below) of more than 200 g/m²/day, preferably breathable with a MVTR of 800 g/m²/day or more preferably 1200 to (inclusive) 1400 g/m²/day, even more preferably breathable with a MVTR of at least 1500 g/m²/day, up to 2100 g/m²/day (inclusive), and most preferably the coating (e.g., the elastomeric polymer) is highly breathable with a MVTR of 2100 g/m²/day or more.

The coating or shell herein is typically thin; preferably it has an average caliper (thickness) between 1 micron (µm) and 100 microns, preferably from 1 micron to 50 microns, more preferably from 1 micron to 20 microns or even from 2 to 20 microns or even from 2 to 10 microns, as can be determined by the method described herein.

The coating or shell is preferably uniform in caliper and/or shape. Preferably, the average caliper is such that the ratio of the smallest to largest caliper is from 1:1 to 1:5, preferably from 1:1 to 1:3, or even 1:1 to 1:2, or even 1:1 to 1:1.5.

Preferably, the film-forming elastomeric polymers are thermoplastic film-forming elastomeric polymers.

The elastomeric polymers herein are non water-absorbing. They typically absorb less than 1.0 g/g water or saline or synthetic urine, preferably even less than 0.5g/g, or even less than 0.1 g/g, as may be determined by the method described herein.

The elastomeric polymer may be a polymer with at least one glass transition temperature of below 60°C; preferred may be that the elastomeric polymer is a block copolymer, whereby at least one segment or block of the copolymer has a Tg below room temperature (i.e., below 25°C; this is said to be the soft segment or soft block) and at least one segment or block of the copolymer that has a Tg above room temperature (and this is said to be the hard segment or hard block), as described in more detail below. The Tg's, as referred to herein, may be measured by methods known in the art, such as Differential Scanning Calorimetry (DSC) to measure the change in specific heat that a material undergoes upon heating. The DSC measures the energy required to maintain the temperature of a sample of the elastomeric polymer to be the same as the temperature of the inert reference material (e.g., Indium). A Tg is determined from the midpoint of the endothermic change in the slope of the baseline. The Tg values are reported from the second heating cycle so that any residual solvent in the sample is removed.

Preferably, the water-absorbing material comprises particles with a coating that comprises one or more film-forming elastomeric polymers with at least one Tg of less than 60°C, whereby said material has a shell impact parameter, which is defined as the (Average wet secant elastic modulus at 400% elongation) * (Relative Weight of said elastomeric polymer compared to the total weight of the water-absorbing material) of 0.03 MPa to 0.6 MPa, preferably 0.07 MPa to 0.45 MPa, more preferably of 0.1 to 0.35 MPa.

The relative weight percentage of the elastomeric polymer above may be determined by, for example, the pulsed NMR method described herein.

In a preferred embodiment, the water-absorbing material comprises elastomeric polymers, present in the coating of the particles thereof, which are typically present at a weight percentage of (by weight of the water-absorbing material) of 0.1% to 25%, or more preferably 0.5 to 15% or even more preferably to 10%, or even more preferably up to 5%. The skilled person would know the suitable methods to determine this. For example, for water-absorbing materials comprising elastomeric polymers with at least one glass transition temperature (Tg) of less than 60°C or less, the NMR method described herein below may be used.

In order to impart desirable properties to the elastomeric polymer, additionally fillers such as particulates, oils, solvents, plasticizers, surfactants, dispersants may be optionally incorporated.

For fast wetting it is preferable that the polymer is hydrophilic.

The elastomeric polymer is preferably applied by the coating processes described herein, by use of a solution or dispersion thereof. Such solutions and dispersions can be prepared using water and/or any suitable organic solvent, for example, acetone, isopropanol, tetrahydrofuran, methyl ethyl ketone, dimethyl sulfoxide, dimethylformamide, chloroform, ethanol, methanol and mixtures thereof.

In a preferred embodiment the polymer is applied in the form of a, preferably aqueous, dispersion and in a more preferred embodiment the polymer is applied as an aqueous dispersion of a polyurethane, such as the preferred polyurethanes described below.

The synthesis of polyurethanes and the preparation of polyurethane dispersions is well described, for example, in Ullmann's Encyclopedia of Industrial Chemistry, Sixth Edition, 2000 Electronic Release.

Polymers can also be blended prior to coating by blending their respective solutions or their respective dispersions. In particular, polymers that do not fulfil the elastic criteria or permeability criteria by themselves can be blended with polymers that do fulfil these criteria and yield a blend that is suitable for coating herein.

In a preferred embodiment the polymer is in the form of an aqueous dispersion and in a more preferred embodiment the polymer is an aqueous dispersion of a polyurethane.

The polyurethane is preferably hydrophilic and in particular surface hydrophilic. The surface hydrophilicity may be determined by methods known to those skilled in the art. In a preferred execution, the hydrophilic polyurethanes are materials that are wetted by the liquid that is to be absorbed (0.9% saline; urine). They may be characterized by a contact angle that is less than 90 degrees. Contact angles can, for example, be measured with the Video-based contact angle measurement device, Krüss G10 - G1041, available from Kruess, Germany or by other methods known in the art.

In a preferred embodiment, the hydrophilic properties are achieved as a result of the polyurethane comprising hydrophilic polymer blocks, for example, polyether groups having a fraction of groups derived from ethylene glycol (CH₂CH₂O) or from 1,4-butanediol (CH₂CH₂CH₂CH₂O) or from propylene glycol (CH₂CH₂CH₂O), or mixtures thereof. Polyetherpolyurethanes are therefore preferred film-forming polymers. The hydrophilic blocks can be constructed in the manner of comb polymers where parts of the side chains or all side chains are hydrophilic polymeric blocks. But the hydrophilic blocks can also be constituents of the main chain (i.e., of the polymer's backbone). A preferred embodiment utilizes polyurethanes where at least the predominant fraction of the hydrophilic polymeric blocks is present in the form of side chains. The side chains can in turn be block copolymers such as poly(ethylene glycol)-co-poly(propylene glycol).

It is further possible to obtain hydrophilic properties for the polyurethanes through an elevated fraction of ionic groups, preferably carboxylate, sulfonate, phosphonate or ammonium groups. The ammonium groups may be protonated or alkylated tertiary or quarternary groups. Carboxylates, sulfonates, and phosphates may be present as alkalimetal or ammonium salts. Suitable ionic groups and their respective precursors are, for example,, described in "Ullmanns Encyclopädie der technischen Chemie", 4th Edition, Volume 19, p. 311-313 and are furthermore described in DE-A 1 495 745 and WO 03/050156.

The hydrophilicity of the preferred polyurethanes facilitates the penetration and dissolution of water into the water-absorbing polymeric particles which are enveloped by the film-forming polymer. The coatings with these preferred polyurethanes are notable for the fact that the mechanical properties are not excessively impaired even in the moist state, despite the hydrophilicity.

Preferred film forming polymers have two or more glass transition temperatures (determined by DSC). Ideally, the polymers used exhibit the phenomenon of phase separation, i.e., they contain two or more different blocks of low and high Tg side by side in the polymer (Thermoplastic Elastomers: A Comprehensive Review, eds. Legge, N.R., Holden, G., Schroeder, H.E., 1987, chapter 2). However, the measurement of Tg may in practice be very difficult in cases when several Tg's are close together or for other experimental reasons.

Especially preferred polyurethanes, herein comprise one or more phase-separating block copolymers, having a weight average molecular weight Mw of at least 5 kg/mol, preferably at least 10 kg/mol and higher.

In one embodiment such a block copolymer has at least a first polymerized homopolymer segment (block) and a second polymerized homopolymer segment (block), polymerized with one another, whereby preferably the first (soft) segment has a Tg₁ of less than 25°C or even less than 20°C, or even less than 0°C, and the second (hard) segment has a Tg₂ of at least 50°C, or of 55°C or more, preferably 60°C or more or even 70°C or more.

In another embodiment, especially with polyurethanes, such a block copolymer has at least a first polymerized heteropolymer segment (block) and a second polymerized heteropolymer segment (block), polymerized with one another, whereby preferably the first (soft) segment has a Tg₁ of less than 25°C or even less than 20°C, or even less than 0°C, and the second (hard) segment has a Tg₂ of at least 50°C, or of 55°C or more, preferably 60°C or more or even 70°C or more.

In one embodiment the total weight average molecular weight of the hard second segments (with a Tg of at least 50°C) is preferably at least 28 kg/mol, or even at least 45 kg/mol.

The preferred weight average molecular weight of a first (soft) segment (with a Tg of less than 25°C) is at least 500 g/mol, preferably at least 1000 g/mol or even at least 2000 g/mol, but preferably less than 8000 g/mol, preferably less than 5000 g/mol.

However, the total of the first (soft) segments is typically 20% to 95% by weight of the total block copolymer, or even from 20% to 85% or more preferably from 30% to 75% or even from 40% to 70% by weight. Furthermore, when the total weight level of soft segments is more than 70%, it is even more preferred that an individual soft segment has a weight average molecular weight of less than 5000 g/mol.

It is well understood by those skilled in the art that "polyurethanes" is a generic term used to describe polymers that are obtained by reacting di- or polyisocyanates with at least one di- or polyfunctional "active hydrogen-containing" compound. "Active hydrogen containing" means that the di- or polyfunctional compound has at least 2 functional groups which are reactive toward isocyanate groups (also referred to as reactive groups), e.g., hydroxyl groups, primary and secondary amino groups and mercapto (SH) groups.

It also is well understood by those skilled in the art that polyurethanes also include allophanate, biuret, carbodiimide, oxazolidinyl, isocyanurate, uretdione, and other linkages in addition to urethane and urea linkages.

In one embodiment the block copolymers useful herein are preferably polyether urethanes and polyester urethanes. Especially preferred are polyether urethanes comprising polyalkylene glycol units, especially polyethylene glycol units or poly(tetramethylene glycol) units.

As used herein, the term "alkylene glycol" includes both alkylene glycols and substituted alkylene glycols having 2 to 10 carbon atoms, such as ethylene glycol, 1,3-propylene glycol, 1,2-propylene glycol, 1,2-butylene glycol, 1,3-butylene glycol, 1,4-butylene glycol, styrene glycol and the like.

The polyurethanes used herein are generally obtained by reaction of polyisocyanates with active hydrogen-containing compounds having two or more reactive groups. These include :
a) high molecular weight compounds having a molecular weight in the range of preferably 300 to 100 000 g/mol especially from 500 to 30 000 g/mol
b) low molecular weight compounds and
c) compounds having polyether groups, especially polyethylene oxide groups or polytetrahydrofuran groups and a molecular weight in the range from 200 to 20 000 g/mol, the polyether groups in turn having no reactive groups.
These compounds can also be used as mixtures.

Suitable polyisocyanates have an average of about two or more isocyanate groups, preferably an average of about two to about four isocyanate groups and include aliphatic, cycloaliphatic, araliphatic, and aromatic polyisocyanates, used alone or in mixtures of two or more. Diisocyanates are more preferred. Especially preferred are aliphatic and cycloaliphatic polyisocyanates, especially diisocyanates.

Specific examples of suitable aliphatic diisocyanates include alpha, omega-alkylene diisocyanates having from 5 to 20 carbon atoms, such as hexamethylene-1,6-diisocyanate, 1,12-dodecane diisocyanate, 2,2,4-trimethylhexamethylene diisocyanate, 2,4,4-trimethyl-hexamethylene diisocyanate, 2-methyl-1,5-pentamethylene diisocyanate, and the like. Polyisocyanates having fewer than 5 carbon atoms can be used but are less preferred because of their high volatility and toxicity. Preferred aliphatic polyisocyanates include hexamethylene-1,6-diisocyanate, 2,2,4-trimethyl-hexamethylene diisocyanate, and 2,4,4-trimethyl-hexamethylene diisocyanate.

Specific examples of suitable cycloaliphatic diisocyanates include dicyclohexylmethane diisocyanate, (commercially available as Desmodur^{®} W from Bayer Corporation), isophorone diisocyanate, 1,4-cyclohexane diisocyanate, 1,3-bis(isocyanatomethyl) cyclohexane, and the like. Preferred cycloaliphatic diisocyanates include dicyclohexylmethane diisocyanate and isophorone diisocyanate.

Specific examples of suitable araliphatic diisocyanates include m-tetramethyl xylylene diisocyanate, p-tetramethyl xylylene diisocyanate, 1,4-xylylene diisocyanate, 1,3-xylylene diisocyanate, and the like. A preferred araliphatic diisocyanate is tetramethyl xylylene diisocyanate.

Examples of suitable aromatic diisocyanates include 4,4'-diphenylmethane diisocyanate, toluene diisocyanate, their isomers, naphthalene diisocyanate, and the like. A preferred aromatic diisocyanate is toluene diisocyanate and 4,4'-diphenylmethane diisocyanate.

Examples of high molecular weight compounds a) having 2 or more reactive groups are such as polyester polyols and polyether polyols, as well as polyhydroxy polyester amides, hydroxyl-containing polycaprolactones, hydroxyl-containing acrylic copolymers, hydroxyl-containing epoxides, polyhydroxy polycarbonates, polyhydroxy polyacetals, polyhydroxy polythioethers, polysiloxane polyols, ethoxylated polysiloxane polyols, polybutadiene polyols and hydrogenated polybutadiene polyols, polyacrylate polyols, halogenated polyesters and polyethers, and the like, and mixtures thereof. The polyester polyols, polyether polyols, polycarbonate polyols, polysiloxane polyols, and ethoxylated polysiloxane polyols are preferred. Particular preference is given to polyesterpolyols, polycarbonate polyols and polyalkylene ether polyols. The number of functional groups in the aforementioned high molecular weight compounds is preferably on average in the range from 1.8 to 3 and especially in the range from 2 to 2.2 functional groups per molecule.

Polyether polyols are obtained in known manner by the reaction of a starting compound that contain reactive hydrogen atoms, such as water or the diols set forth for preparing the polyester polyols, and alkylene glycols or cyclic ethers, such as ethylene glycol, propylene glycol, butylene glycol, styrene glycol, ethylene oxide, propylene oxide, 1,2-butylene oxide, 2,3-butylene oxide, oxetane, tetrahydrofuran, epichlorohydrin, and the like, and mixtures thereof. Preferred polyethers include poly(ethylene glycol), poly(propylene glycol), polytetrahydrofuran, and co [poly(ethylene glycol)-poly(propylene glycol)]. Polyethylenglycol and Polypropyleneglycol can be used as such or as physical blends. In case that propyleneoxide and ethylenoxide are copolymerized, these polypropylene-co-polyethylene polymers can be used as random polymers or blockcopolymers.

In one embodiment, the polyetherpolyol is a constituent of the main polymer chain.

In another embodiment, the polyetherol is a terminal group of the main polymer chain.

In yet another embodiment, the polyetherpolyol is a constituent of a side chain which is comb-like attached to the main chain. An example of such a monomer is Tegomer D-3403 (Degussa).

Examples of low molecular weight compounds b) having two reactive functional groups are the diols such as alkylene glycols and other diols mentioned above in connection with the preparation of polyesterpolyols. They also include amines such as diamines and polyamines which are among the preferred compounds useful in preparing the aforesaid polyesteramides and polyamides. Suitable diamines and polyamines include 1,2-diaminoethane, 1,6-diaminohexane, 2-methyl-1,5-pentanediamine, 2,2,4-trimethyl-1,6-hexanediamine, 1,12-diaminododecane, 2-aminoethanol, 2-[(2-aminoethyl)amino]-ethanol, piperazine, 2,5-dimethylpiperazine, 1-amino-3-aminomethyl-3,5,5-trimethylcyclohexane (isophorone diamine or IPDA), bis-(4-aminocyclohexyl)-methane, bis-(4-amino-3-methyl-cyclohexyl)-methane, 1,4-diaminocyclohexane, 1,2-propylenediamine, hydrazine, urea, amino acid hydrazides, hydrazides of semicarbazidocarboxylic acids, bis-hydrazides and bis-semicarbazides, diethylene triamine, triethylene tetramine, tetraethylene pentamine, pentaethylene hexamine, N,N,N-tris-(2-aminoethyl)amine, N-(2-piperazinoethyl)-ethylene diamine, N,N'-bis-(2-aminoethyl)-piperazine, N,N,N'-tris-(2-aminoethyl)ethylene diamine, N-[N-(2-aminoethyl)-2-aminoethyl]-N'-(2-aminoethyl)-piperazine, N-(2-aminoethy)-N'-(2-piperazinoethyl)-ethylene diamine, N,N-bis-(2-aminoethyl)-N-(2-piperazinoethyl)amine, N,N-bis-(2-piperazinoethyl)amine, polyethylene imines, iminobispropylamine, guanidine, melamine, N-(2-aminoethyl)-1,3-propane diamine, 3,3'-diaminobenzidine, 2,4,6-triaminopyrimidine, polyoxypropylene amines, tetrapropylenepentamine, tripropylenetetramine, N,N-bis-(6-aminohexyl)amine, N,N'-bis-(3-aminopropyl)ethylene diamine, and 2,4-bis-(4'-aminobenzyl)-aniline, and the like, and mixtures thereof. Preferred diamines and polyamines include 1-amino-3-aminomethyl-3,5,5-trimethylcyclohexane (isophorone diamine or IPDA), bis-(4-aminocyclohexyl)-methane, bis-(4-amino-3-methylcyclohexyl)-methane, ethylene diamine, diethylene triamine, triethylene tetramine, tetraethylene pentamine, and pentaethylene hexamine, and the like, and mixtures thereof. Other suitable diamines and polyamines, for example,, include Jeffamine^{®} D-2000 and D-4000, which are amine-terminated polypropylene glycols differing only by molecular weight, and Jeffamine^{®} XTJ-502, T 403, T 5000, and T 3000 which are amine terminated polyethyleneglycols, amine terminated co-polypropylene-polyethylene glycols, and triamines based on propoxylated glycerol or trimethylolpropane and which are available from Huntsman Chemical Company.

The poly(alkylene glycol) may be part of the polymer main chain. In any event, it is attached to the main chain in comb-like shape as a side chain.

The polyurethane comprises poly(alkylene glycol) side chains, preferably in amount from 10 wt.% to 90 wt.%, preferably about 12 wt% to about 80 wt.%, preferably about 15 wt.% to about 60 wt.%, and more preferably about 20 wt.% to about 50 wt.%, of poly(alkylene glycol) units in the final polyurethane on a dry weight basis. At least about 50 wt.%, preferably at least about 70 wt.%, and more preferably at least about 90 wt.% of the poly(alkylene glycol) side-chain units comprise poly(ethylene glycol), and the remainder of the side-chain poly-(alkylene glycol) units can comprise alkylene glycol and substituted alkylene glycol units having from 3 to about 10 carbon atoms. The term "final polyurethane" means the polyurethane used for coating the water-absorbing polymeric particles.

Preferably the amount of the side-chain units is (i) at least about 30 wt.% when the molecular weight of the side-chain units is less than about 600 g/mol, (ii) at least about 15 wt.% when the molecular weight of the side-chain units is from about 600 to about 1000 g/mol, and (iii) at least about 12 wt.% when the molecular weight of said side-chain units is more than about 1000 g/mol. Mixtures of active hydrogen-containing compounds having such poly(alkylene glycol) side chains can be used with active hydrogen-containing compounds not having such side chains.

These side chains can be incorporated in the polyurethane by replacing a part or all of the aforementioned high molecular diols a) or low molecular compounds b) by compounds c) having at least two reactive functional groups and a polyether group, preferably a polyalkylene ether group, more preferably a polyethylene glycol group that has no further reactive group.

For example, active hydrogen-containing compounds having a polyether group, in particular a poly(alkylene glycol) group, include diols having poly(ethylene glycol) groups such as those described in U.S. Pat. No. 3,905,929 . Further, U.S. Pat. No. 5,700,867 teaches methods for incorporation of poly(ethylene glycol) side chains at col. 4, line 3.5 to col. 5, line 4.5. A preferred active hydrogen-containing compound having poly(ethylene glycol) side chains is trimethylol propane mono (polyethylene oxide methyl ether), available as Tegomer D-3403 from Degussa-Goldschmidt.

Preferably, the polyurethanes to be used herein also have reacted therein at least one active hydrogen-containing compound not having said side chains and typically ranging widely in molecular weight from 50 to 10000 g/mol, preferably 200 to 6000 g/mol, and more preferably 300 to 3000 g/mol. Suitable active hydrogen-containing compounds not having said side chains include any of the amines and polyols described herein as compounds a) and b).

According to one preferred embodiment herein, the active hydrogen compounds are chosen to provide less than about 25 wt.%, more preferably less than about 15 wt.% and most preferably less than about 5 wt.% poly(ethylene glycol) units in the backbone (main chain) based upon the dry weight of final polyurethane, since such main-chain poly(ethylene glycol) units tend to cause swelling of polyurethane particles in the waterborne polyurethane dispersion and also contribute to lower in use tensile strength of articles made from the polyurethane dispersion.

The preparation of polyurethanes having polyether side chains is known to one skilled in the art and is extensively described, for example" in US 2003/0195293 .

Advantageous polyurethanes herein are obtained by first preparing prepolymers having isocyanate end groups, which are subsequently linked together in a chain-extending step. The linking together can be through water or through reaction with a compound having at least one crosslinkable functional group.

The prepolymer is obtained by reacting one of the above-described isocyanate compounds with an active hydrogen compound. Preferably the prepolymer is prepared from the above mentioned polyisocyanates, at least one compound c) and optionally at least one further active hydrogen compound selected from the compounds a) and b).

In one embodiment, the ratio of isocyanate to active hydrogen in the compounds forming the prepolymer typically ranges from 1.3/1 to 2.5/1, preferably from 1.5/1 to 2.1/1, and more preferably from 1.7/1 to 2/1.

The polyurethane may additionally contain functional groups which can undergo further crosslinking reactions and which can optionally render them self-crosslinkable.

Compounds having at least one additional crosslinkable functional group include those having carboxylic, carbonyl, amine, hydroxyl, and hydrazide groups, and the like, and mixtures of such groups. The typical amount of such optional compound is up to 1 milliequivalent, preferably from 0.05 to 0.5 milliequivalent, and more preferably from 0.1 to 0.3 milliequivalent per gram of final polyurethane on a dry weight basis.

The preferred monomers for incorporation into the isocyanate-terminated prepolymer are hydroxy-carboxylic acids having the general formula (HO)ₓQ(COOH)_{y} wherein Q is a straight or branched hydrocarbon radical having 1 to 12 carbon atoms, and x and y are 1 to 3. Examples of such hydroxy-carboxylic acids include citric acid, dimethylolpro- panoic acid (DMPA), dimethylol butanoic acid (DMBA), glycolic acid, lactic acid, malic acid, dihydroxymalic acid, tartaric acid, hydroxypivalic acid, and the like, and mixtures thereof. Dihydroxy-carboxylic acids are more preferred with dimethylolpropanoic acid (DMPA) being most preferred.

Other suitable compounds providing crosslinkability include thioglycolic acid, 2,6-dihydroxybenzoic acid, and the like, and mixtures thereof.

Optional neutralization of the prepolymer having pendant carboxyl groups converts the carboxyl groups to carboxylate anions, thus having a water-dispersibility enhancing effect. Suitable neutralizing agents include tertiary amines, metal hydroxides, ammonia, and other agents well known to those skilled in the art.

As a chain extender, at least one of water, an inorganic or organic polyamine having an average of about 2 or more primary and/or secondary amine groups, polyalcohols, ureas, or combinations thereof is suitable herein. Suitable organic amines for use as a chain extender include diethylene triamine (DETA), ethylene diamine (EDA), meta-xylylenediamine (MXDA), aminoethyl ethanolamine (AEEA), 2-methyl pentane diamine, and the like, and mixtures thereof. Also suitable are propylene diamine, butylene diamine, hexamethylene diamine, cyclohexylene diamine, phenylene diamine, tolylene diamine, 3,3-dichlorobenzidene, 4,4'-methylene-bis-(2-chloroaniline), 3,3-dichloro- 4,4-diamino diphenylmethane, sulfonated primary and/or secondary amines, and the like, and mixtures thereof. Suitable inorganic and organic amines include hydrazine, substituted hydrazines, and hydrazine reaction products, and the like, and mixtures thereof. Suitable polyalcohols include those having from 2 to 12 carbon atoms, preferably from 2 to 8 carbon atoms, such as ethylene glycol, diethylene glycol, neopentyl glycol, butanediols, hexanediol, and the like, and mixtures thereof. Suitable ureas include urea and its derivatives, and the like, and mixtures thereof. Hydrazine is preferred and is most preferably used as a solution in water. The amount of chain extender typically ranges from about 0.5 to about 0.95 equivalents based on available isocyanate.

A degree of branching of the polyurethane may be beneficial, but is not required to maintain a high tensile strength and improve resistance to creep (cf. strain relaxation). This degree of branching may be accomplished during the prepolymer step or the extension step. For branching during the extension step, the chain extender DETA is preferred, but other amines having an average of about two or more primary and/or secondary amine groups may also be used. For branching during the prepolymer step, it is preferred that trimethylol propane (TMP) and other polyols having an average of more than two hydroxyl groups be used. The branching monomers can be present in amounts up to about 4 wt.% of the polymer backbone.

Polyurethanes are preferred film-forming polymers. They can be applied to the water-absorbing polymer particles from solvent or from a dispersion. Particularly preferred are aqueous dispersions.

Particularly suitable elastomeric film-forming polyurethanes are extensively described in the literature references hereinbelow and expressly form part of the subject matter of the present disclosure. Particularly hydrophilic thermoplastic polyurethanes are sold by Noveon, Brecksville, Ohio, under the tradenames of Permax^{®} 120, Permax 200 and Permax 220 and are described in detail in "Proceedings International Waterborne High Solids Coatings, 32, 299, 2004" and were presented to the public in February 2004 at the "International Waterborne, High-Solids, and Powder Coatings Symposium" in New Orleans, USA. The preparation is described in detail in US 2003/0195293.

More particularly, the polyurethanes described can be used in mixtures with each other or with other film-forming polymers, fillers, oils, water-soluble polymers or plasticizing agents in order that particularly advantageous properties may be achieved with regard to hydrophilicity, water perviousness and mechanical properties.

It may be preferred that the coating agent herein comprises fillers to reduce tack such as the commercially available resin Estane 58245-047P and Estane X-1007-040P, available from Noveon Inc., 9911 Brecksville Road, Cleveland, OH 44141-3247, USA.

Alternatively such fillers can be added in order to reduce tack to the dispersions or solutions of suitable elastomeric polymers before application. A typical filler is Aerosil, but other inorganic deagglomeration aids as listed below can also be used.

Preferred polyurethanes for use in the coating agent herein are strain hardening and/or strain crystallizing. Strain Hardening is observed during stress-strain measurements, and is evidenced as the rapid increase in stress with increasing strain. It is generally believed that strain hardening is caused by orientation of the polymer chains in the film producing greater resistance to extension in the direction of drawing.

The coating agent is applied such that the resulting coating layer is preferably thin having an average calliper (thickness) of more than 0.1 µm; preferably the coating layer has an average caliper (thickness) from 1 micron (µm) to 100 microns, preferably from 1 micron to 50 microns, more preferably from 1 micron to 20 microns or even from 2 to 20 microns or even from 2 to 10 microns.

The polymeric coating is preferably applied in an amount of 0.1 - 25 parts by weight of the film-forming polymer (reckoned as solids material) to 100 parts by weight of dry water-absorbing polymeric particles. The amount of film-forming polymer used per 100 parts by weight of water-absorbing polymeric particles is preferably 0.1 - 15 parts by weight, especially 0.5 - 10 parts by weight, more preferably 0.5 - 7 parts by weight, even more preferably 0.5 - 5 parts by weight and in particular 0.5 - 4.5 parts by weight.

Particular preference is given to a water-absorbing material obtained by coating water-absorbing polymeric particles with <5 parts by weight, preferably 0.5 - 4.5 parts by weight, especially 0.5 - 4 parts by weight and more preferably 0.5 - 3 parts by weight of film-forming polymer based on 100 parts by weight of water-absorbing polymeric particles, preferably at temperatures in the range from 0°C to <50°C, preferably from 0°C to <45°C, more preferably from 10°C to <40°C, and most preferably from 15°C to <35°C, and then heat-treating the coated particles at a temperature above 50°C.

### Water-absorbing polymers

The water-absorbing polymers herein are preferably solid, preferably in the form of particles (which includes, for example, particles in the form of flakes, fibers, agglomerates). The water-absorbing polymer particles can be spherical in shape as well as irregularly shaped particles.

Useful herein are in principle all particulate water-absorbing polymers known to one skilled in the art from superabsorbent literature, for example, as described in Modem Superabsorbent Polymer Technology, F.L. Buchholz, A.T. Graham, Wiley 1998. The water-absorbing particles are preferably spherical water-absorbing particles of the kind typically obtained from inverse phase suspension polymerizations; they can also be optionally agglomerated at least to some extent to form larger irregular particles. But most particular preference is given to commercially available irregularly shaped particles of the kind obtainable by current state of the art production processes as is more particularly described herein below by way of example.

Olefinically unsaturated carboxylic acid and anhydride monomers useful herein include the acrylic acids typified by acrylic acid itself, methacrylic acid, α-chloroacrylic acid, α-cyanoacrylic acid, β-methylacrylic acid (crotonic acid), α-phenylacrylic acid, β-acryloxypropionic acid, sorbic acid, α-chlorosorbic acid, angelic acid, cinnamic acid, p-chlorocinnamic acid, β-stearylacrylic acid, itaconic acid, citroconic acid, mesaconic acid, glutaconic acid, aconitic acid, maleic acid, fumaric acid, tricarboxyethylene, and maleic anhydride. Preferred water-absorbing polymers contain carboxyl groups, such as the above-described carboxylic acid/ carboxylate containing groups. These polymers include hydrolyzed starch-acrylonitrile graft copolymers, partially neutralized hydrolyzed starch-acrylonitrile graft copolymers, starch-acrylic acid graft copolymers, partially neutralized starch-acrylic acid graft copolymers, hydrolyzed vinyl acetate-acrylic ester copolymers, hydrolyzed acrylonitrile or acrylamide copolymers, slightly network crosslinked polymers of any of the aforementioned copolymers, polyacrylic acid, and slightly network crosslinked polymers of polyacrylic acid.

The water-absorbing polymers are preferably polymeric particles obtainable by polymerization of a monomer solution comprising:
i) at least one ethylenically unsaturated acid-functional monomer,
ii) at least one crosslinker,
iii) if appropriate one or more ethylenically and/or allylically unsaturated monomers copolymerizable with i) and
iv) if appropriate one or more water-soluble polymers onto which the monomers i), ii) and if appropriate iii) can be at least partially grafted,
   wherein the base polymer obtained thereby is dried, classified and if appropriate is subsequently treated with
v) at least one post-crosslinker (or: surface cross-linker)
before being dried and optionally post-crosslinked (i.e., Surface crosslinked).

Useful monomers i) include, for example, ethylenically unsaturated carboxylic acids, such as acrylic acid, methacrylic acid, maleic acid, fumaric acid, and itaconic acid, or derivatives thereof, such as acrylamide, methacrylamide, acrylic esters and methacrylic esters. Acrylic acid and methacrylic acid are particularly preferred monomers.

The water-absorbing polymers to be used herein are typically crosslinked, i.e., the polymerization is carried out in the presence of compounds having two or more polymerizable groups which can be free-radically copolymerized into the polymer network.

The preparation of a suitable base polymer and also further useful hydrophilic ethylenically unsaturated monomers i) are described in DE-A 199 41 423, EP-A 686 650, WO 01/45758 and WO 03/14300.

The acid groups of the base polymers obtained are preferably 30 - 100 mol%, more preferably 65 - 90 mol% and most preferably 72 - 85 mol% neutralized, for which the customary neutralizing agents can be used.

Neutralization can be carried out after polymerization, at the base polymer stage. But it is also possible to neutralize up to 40 mol%, preferably from 10 to 30 mol% and more preferably from 15 to 25 mol% of the acid groups before polymerization by adding a portion of the neutralizing agent to the monomer solution and to set the desired final degree of neutralization only after polymerization, at the base polymer stage.

Most preferably, the water-absorbing polymers comprise from about 50% to 95% (mol percentage), preferably about 75 mol% neutralized, (slightly) crosslinked, polyacrylic acid (i.e., poly (sodium acrylate/acrylic acid)).

The neutralized base polymer is then dried with a belt, fluidized bed, tower dryer or drum dryer until the residual moisture content is preferably below 13% by weight, especially below 8% by weight and most preferably below 4% by weight, the water content being determined according to EDANA's recommended test method No. 430.2-02 "Moisture content" (EDANA = European Disposables and Nonwovens Association). The dried base polymer is thereafter ground and sieved, useful grinding apparatus typically include roll mills, pin mills, hammer mills, jet mills or swing mills.

The water-absorbing polymers to be used can be post-crosslinked (surface crosslinked).

Useful post-crosslinkers include compounds comprising two or more groups capable of forming covalent bonds with the carboxylate groups of the polymers. The post-crosslinker is typically used in an amount of about 1.50 wt.% or less, preferably not more than 0.50% by weight, more preferably not more than 0.30% by weight and most preferably in the range from 0.001 % and 0.15% by weight, all percentages being based on the base polymer, as an aqueous solution. It is possible to use a single post-crosslinker from the above selection or any desired mixtures of various post-crosslinkers.

The concentration of the at least one post-crosslinker v) in the aqueous post-crosslinking solution is, for example,, in the range from 1% to 50% by weight, preferably in the range from 1.5% to 20% by weight and more preferably in the range from 2% to 5% by weight, based on the post-crosslinking solution.

It is, however, understood that post-crosslinkers which effect comparable surface-crosslinking results with respect to the final polymer performance may of course be used herein even when the water content of the solution containing such post-crosslinker and optionally a cosolvent is anywhere in the range of >0 to <100% by weight.

The total amount of post-crosslinking solution based on the base polymer is typically in the range from 0.3% to 15% by weight and preferably in the range from 2% to 6% by weight. The practice of post-crosslinking is common knowledge to those skilled in the art and described, for example, in DE-A-12 239 074 and also prior German patent application 102004051242.6.

The water-absorbing polymeric particles can have a particle size distribution in the range from 45 µm to 4000 µm. Particle sizes used in the hygiene sector preferably range from 45 µm to 1000 µm, preferably from 45 - 850 µm, and especially from 100 µm to 850 µm. It is preferable to use water-absorbing polymeric particles having a narrow particle size distribution, especially 100 - 850 µm, or even 100 - 600µm.

Narrow particle size distributions are those in which not less than 80% by weight of the particles, preferably not less than 90% by weight of the particles and most preferably not less than 95% by weight of the particles are within the selected range; this fraction can be determined using the familiar sieve method of EDANA 420.2-02 "Particle Size Distribution". Selectively, optical methods can be used as well, provided these are calibrated against the accepted sieve method of EDANA.

Preferred narrow particle size distributions have a span of not more than 700 µm, more preferably of not more than 600 µm, and most preferably of less than 400 µm. Span here refers to the difference between the coarse sieve and the fine sieve which bound the distribution. The coarse sieve is not coarser than 850 µm and the fine sieve is not finer than 45 µm. Particle size ranges which are preferred herein are, for example, fractions of 150 - 600 µm (span: 450 µm), of 200 - 700 µm (span: 500 µm), of 150 - 500 µm (span: 350 µm), of 150 - 300 µm (span: 150 µm), of 300 - 700 µm (span: 400 µm), of 400 - 800 µm (span: 400 µm), of 100 - 800 µm (span: 700 µm).

### Process

The elastomeric polymer especially the polyurethane can be applied as a solid material, as a hotmelt, as a dispersion, as an aqueous dispersion, as an aqueous solution or as an organic solution to the particles of the water-absorbing addition polymer. The form in which the film-forming polymer, especially the polyurethane is applied to the water-absorbing polymeric particles is preferably as a solution or more preferably as an aqueous dispersion.

Useful solvents for polyurethanes include solvents which make it possible to establish 1 to 40% by weight concentrations of the polyurethane in the respective solvent or mixture. As examples there may be mentioned alcohols, esters, ethers, ketones, amides, and halogenated hydrocarbons like methyl ethyl ketone, acetone, isopropanol, tetrahydrofuran, dimethylformamide, chloroform and mixtures thereof. Solvents which are polar, aprotic and boil below 100°C are particularly advantageous.

Aqueous herein refers to water and also mixtures of water with up to 20% by weight of water-miscible solvents, based on the total amount of solvent. Water-miscible solvents are miscible with water in the desired use amount at 25°C and 1 bar. They include alcohols such as methanol, ethanol, propanol, isopropanol, ethylene glycol, 1,2-propanediol, 1,3-propanediol, ethylene carbonate, glycerol and methoxyethanol and water-soluble ethers such as tetrahydrofuran and dioxane.

It is particularly preferable to affect the coating in a fluidized bed reactor. The water-absorbing particles are introduced as generally customary, depending on the type of the reactor, and are generally coated by spraying with the film-forming polymer as a solid material or preferably as a polymeric solution or dispersion. Aqueous dispersions of the film-forming polymer are particularly preferred for this.

Preferably, the process may involve:
a) spray-coating water-absorbing polymeric particles with an elastomeric film-forming polymer in a fluidized bed reactor, preferably in a continuous process, in the range from 0°C to 50°C, preferably to less than 45°C, and
b) heat-treating the coated particles at a temperature above 50°C.

The polyurethane solution or dispersion applied by spray-coating is preferably very concentrated. For this, the viscosity of this polyurethane mixture must not be too high, or the polyurethane solution or dispersion can no longer be finely dispersed for spraying. Preference is given to a polyurethane solution or dispersion having a viscosity of <500 mPa·s, preferably of <300 mPa·s, more preferably of <100 mPa·s, even more preferably of <10 mPa·s, and most preferably < 5mPa·s (typically determined with a rotary viscometer at a shear rate ≥ 200 rpm for the polyurethane dispersion, and specifically suitable is a Haake rotary viscometer type RV20, system M5, NV.

In embodiments in which other film-forming polymers are used, it is preferred that these exhibit the same viscosities as the polyurethanes when applied.

The concentration of polyurethane in the polyurethane solution or dispersion is generally in the range from 1% to 60% by weight, preferably in the range from 5% to 40% by weight and especially in the range from 10% to 30% by weight. Higher dilutions are possible, but generally lead to longer coating times. A particular advantage of polyurethane dispersions is their relatively low viscosity even at high concentrations and high molecular weights.

Useful fluidized bed reactors include, for example, the fluidized or suspended bed coaters familiar in the pharmaceutical industry. Particular preference is given to the Wurster process and the Glatt-Zeller process and these are described, for example, in "Pharmazeutische Technologie, Georg Thieme Verlag, 2nd edition (1989), pages 412-413" and also in "Arzneiformenlehre, Wissenschaftliche Verlagsbuchandlung mbH, Stuttgart 1985, pages 130-132". Particularly suitable batch and continuous fluidized bed processes on a commercial scale are described in Drying Technology, 20(2), 419-447 (2002).

The feature common to the two processes is that the particles are repeatedly carried in the form of a fluidized bed past the spray device, whereby a very thin and typically very homogeneous coating or shell can be applied. Furthermore, a carrier gas is used at all times and it has to be fed and moved at a sufficiently high rate to maintain fluidization of the particles. As a result, liquids are rapidly vaporized in the apparatus, such as, for example, the solvent (i.e., water) of the dispersion, even at low temperatures, whereby the polymeric particles of the dispersion are precipitated onto the surface of the particles of the absorbent polymer which are to be coated. Useful carrier gases include the inert gases mentioned above and air or dried air or mixtures of any of these gases.

Suitable fluidized bed reactors work according to the principle that the film-forming polymer solution or dispersion is finely atomized and the droplets randomly collide with the water-absorbing polymer particles in a fluidized bed, whereby a substantially homogeneous shell builds up gradually and uniformly after many collisions. The size of the droplets must be inferior to the particle size of the absorbent polymer. Droplet size is determined by the type of nozzle, the spraying conditions, i.e., temperature, concentration, viscosity, pressure and typical droplets sizes are in the range 10 µm to 400 µm. A polymer particle size vs. droplet size ratio of at least 10 is typically observed. Small droplets with a narrow size distribution are favourable. The droplets of the atomized polymeric dispersion or solution are introduced either concurrently with the particle flow or from the side into the particle flow, and may also be sprayed from the top onto a fluidized bed. In this sense, other apparatus and equipment modifications which comply with this principle and which are likewise capable of building up fluidized beds are perfectly suitable for producing such effects.

One embodiment, for example, is a cylindrical fluidized bed batch reactor, in which the water-absorbing polymer particles are transported upwards by a carrier-gas stream at the outer walls inside the apparatus and from one or more positions a film-forming polymer spray is applied from the side into this fluidized bed, whereas in the middle zone of the apparatus, in which there is no carrier gas stream at all and where the particles fall down again, a cubic agitator is moving and redistributing the entire fluidized particle bed.

Other embodiments, for example, may be Schuggi mixers, turbolizers or plowshare mixers which can be used alone or preferably as a battery of plural consecutive units. If such a mixer is used alone, the water-absorbing polymer may have to be fed multiple times through the apparatus to become homogeneously coated. If two or more of such apparatus are set up as consecutive units then one pass may be sufficient.

Also continuous or batch-type spray-mixers of the Telschig-type are used in which the spray hits free falling particles in-flight, the particles being repeatedly exposed to the spray. Suitable mixers are described in Chemie-Technik, 22 (1993), Nr. 4, p. 98 ff.

Highly preferred may be to use a continuous fluidized bed process whereby the spray is operated in top or bottom-mode. A suitable apparatus is, for example, described in US 5,211,985. Suitable apparatus are available also, for example, from Glatt Maschinen- und Apparatebau AG (Switzerland) as series GF (continuous fluidized bed) and as ProCell® spouted bed. The spouted bed technology uses a simple slot instead of a screen bottom to generate the fluidized bed and is particularly suitable for materials which are difficult to fluidize.

Two-material nozzles are particularly preferred.

The process herein preferably utilizes Wurster Coaters. Examples for such coaters are PRECISION COATERS™ available from GEA-Aeromatic Fielder AG (Switzerland) and are accessible at Coating Place Inc. (Wisconsin, USA).

It is advantageous that the fluidized bed gas stream which enters from below is likewise chosen such that the total amount of the water-absorbing polymeric particles is fluidized in the apparatus. The gas velocity for the fluidized bed is above the minimum fluidization velocity (measurement method described in Kunii and Levenspiel "Fluidization engineering" 1991) and below the terminal velocity of water-absorbing polymer particles, preferably 10% above the minimum fluidization velocity. The gas velocity for the Wurster tube is above the terminal velocity of water-absorbing polymer particles, usually below 100 m/s, preferably 10% above the terminal velocity.

The gas stream acts to vaporize the water, or the solvents. The coating conditions of gas stream and temperature are chosen so that the relative humidity or vapor saturation at the exit of the gas stream is in the range from 10% to 90%, preferably from 10% to 80%, or preferably from 10% to 70% and especially from 30% to 60%, based on the equivalent absolute humidity prevailing in the carrier gas at the same temperature or, if appropriate, the absolute saturation vapor pressure.

The coating takes typically place at a product and/or carrier gas temperature in the range from 0°C to 50°C, preferably at 5 - 45°C, especially 10 - 40°C and most preferably 15 - 35°C.

A deagglomerating aid may be added before the heat-treating step to the particles to be coated or preferably which have already been coated. A deagglomerating aid would be known by those skilled in the art to be, for example, a finely divided water-insoluble salt selected from organic and inorganic salts and mixtures thereof, and also waxes and surfactants. A water-insoluble salt refers herein to a salt which at a pH of 7 has a solubility in water of less than 5 g/l, preferably less than 3 g/l, especially less than 2 g/l and most preferably less than 1 g/l (at 25°C and 1 bar). The use of a water-insoluble salt can reduce the tackiness due to the film-forming polymer, especially the polyurethane which appears in the course of heat-treating.

The water-insoluble salts are used as a solid material or in the form of dispersions, preferably as an aqueous dispersion. Solids are typically jetted into the apparatus as fine dusts by means of a carrier gas. The dispersion is preferably applied by means of a high speed stirrer by preparing the dispersion from solid material and water in a first step and introducing it in a second step rapidly into the fluidized bed preferably via a nozzle. Preferably both steps are carried out in the same apparatus. The aqueous dispersion can if appropriate be applied together with the polyurethane (or other film-forming polymer) or as a separate dispersion via separate nozzles at the same time as the polyurethane or at different times from the polyurethane. It is particularly preferable to apply the deagglomerating aid after the film-forming polymer has been applied and before the subsequent heat-treating step.

Suitable cations in the water-insoluble salt are, for example,, Ca²⁺, Mg²⁺, Al³⁺, Sc³⁺, Y³⁺, Ln³⁺ (where Ln denotes lanthanoids), Ti⁴⁺, Zr⁴⁺, Li⁺, K⁺, Na⁺ or Zn²⁺. Suitable inorganic anionic counterions are, for example, carbonate, sulfate, bicarbonate, orthophosphate, silicate, oxide or hydroxide. When a salt occurs in various crystal forms, all crystal forms of the salt shall be included. The water-insoluble inorganic salts are preferably selected from calcium sulfate, calcium carbonate, calcium phosphate, calcium silicate, calcium fluoride, apatite, magnesium phosphate, magnesiumhydroxide, magnesium oxide, magnesium carbonate, dolomite, lithium carbonate, lithium phosphate, zinc oxide, zinc phosphate, oxides, hydroxides, carbonates and phosphates of the lanthanoids, sodium lanthanoid sulfate, scandium sulfate, yttrium sulfate, lanthanum sulfate, scandium hydroxide, scandium oxide, aluminum oxide, hydrated aluminum oxide and mixtures thereof. Apatite refers to fluoroapatite, hydroxyl apatite, chloroapatite, carbonate apatite and carbonate fluoroapatite. Of particular suitability are calcium and magnesium salts such as calcium carbonate, calcium phosphate, magnesium carbonate, calcium oxide, magnesium oxide, calcium sulfate and mixtures thereof. Amorphous or crystalline forms of aluminum oxide, titanium dioxide and silicon dioxide are also suitable. These deagglomerating aids can also be used in their hydrated forms. Useful deagglomerating aids further include many clays, talcum and zeolites. Silicon dioxide is preferably used in its amorphous form, for example, as hydrophilic or hydrophobic Aerosil^{®}, but selectively can also be used as aqueous commercially available silica sol, such as, for example, Levasil® Kiselsole (H.C. Starck GmbH), which have particle sizes in the range 5 - 75 nm.

The average particle size of the fmely divided water-insoluble salt is typically less than 200 µm, preferably less than 100 µm, especially less than 50 µm, more preferably less than 20 µm, even more preferably less than 10 µm and most preferably in the range of less than 5 µm. Fumed silicas are often used as even finer particles, e.g., less than 50 nm, preferably less than 30 nm, even more preferably less than 20 nm primary particle size.

The finely divided water-insoluble salt may be used in an amount in the range from 0.001% to 20% by weight, preferably less than 10% by weight, especially in the range from 0.001 % to 5% by weight, more preferably in the range from 0.001 % to 2% by weight and most preferably between 0.001 and 1% by weight, based on the weight of the water-absorbing polymer.

In lieu of or in addition to the above inorganic salts it is also possible to use other known deagglomerating aids, examples being waxes and preferably micronized or preferably partially oxidized polyethylenic waxes, which can likewise be used in the form of an aqueous dispersion. Such waxes are described in EP 0 755 964, which is hereby expressly incorporated herein by reference.

Furthermore, to achieve deagglomeration, a second coating with a dispersion of another polymer of high Tg (>50 °C) can be carried out.

Useful deagglomerating aids further include stearic acid, stearates - for example: magnesium stearate, calcium stearate, zinc stearate, aluminum stearate, and furthermore polyoxyethylene-20-sorbitan monolaurate and also polyethylene glycol 400 monostearate.

Useful deagglomerating aids likewise include surfactants. A surfactant can be used alone or mixed with one of the abovementioned deagglomerating aids, preferably a water-insoluble salt.

The addition can take place together with the polyurethane, before the addition of the polyurethane or after the addition of the polyurethane. In general, it can be added before heat-treating. The surfactant can further be applied during the post-crosslinking operation.

Useful surfactants include nonionic, anionic and cationic surfactants and also mixtures thereof. The water-absorbing material preferably comprises nonionic surfactants. Useful nonionic surfactants include, for example,, sorbitan esters, such as the mono-, di- or triesters of sorbitans with C₈-C₁₈-carboxylic acids such as lauric, palmitic, stearic and oleic acids; polysorbates; alkylpolyglucosides having 8 to 22 and preferably 10 to 18 carbon atoms in the alkyl chain and 1 to 20 and preferably 1.1 to 5 glucoside units; N-alkylglucamides; alkylamine alkoxylates or alkylamide ethoxylates; alkoxylated C₈-C₂₂-alcohols such as fatty alcohol alkoxylates or oxo alcohol alkoxylates; block polymers of ethylene oxide, propylene oxide and/or butylene oxide; alkylphenol ethoxylates having C₆-C₁₄-alkyl chains and 5 to 30 mol of ethylene oxide units.

The amount of surfactant is generally in the range from 0.01% to 0.5% by weight, preferably less than 0.1% by weight and especially below 0.05% by weight, based on the weight of the water-absorbing material.

Heat-treating takes typically place at temperatures above 50°C, preferably in a temperature range from 100 to 200°C, especially 120 - 160°C. Without wishing to be bound by theory, the heat-treating causes the applied film-forming polymer, preferably polyurethane, to flow and form a polymeric film whereby the polymer chains are entangled. The duration of the heat-treating is dependent on the heat-treating temperature chosen and the glass transition and melting temperatures of the film-forming polymer. In general, a heat-treating time in the range from 30 minutes to 120 minutes will be found to be sufficient. However, the desired formation of the polymeric film can also be achieved when heat-treating for less than 30 minutes, for example, in a fluidized bed dryer. Longer times are possible, of course, but especially at higher temperatures can lead to damage in the polymeric film or to the water-absorbing material.

The heat-treating is carried out, for example, in a downstream fluidized bed dryer, a tunnel dryer, a tray dryer, a tower dryer, one or more heated screws or a disk dryer or a Nara® dryer. Heat-treating is preferably done in a fluidized bed reactor and more preferably directly in the Wurster Coater.

The heat-treating can take place on trays in forced air ovens. In this case it is desirable to treat the coated polymer with a deagglomerating aid before heat-treating. Alternatively, the tray can be antistick coated and the coated polymer then placed on the tray as a monoparticulate layer in order that sintering together may be avoided.

In one embodiment, for the process steps of coating, heat-treating, and cooling, it may be possible to use air or dried air in each of these steps.

In other embodiments, an inert gas may be used in one or more of these process steps.

In yet another embodiment, one can use mixtures of air and inert gas in one or more of these process steps.

The heat-treating is preferably carried out under inert gas. It is particularly preferable that the coating step be carried out under inert gas as well. It is very particularly preferable when the concluding cooling phase is carried out under protective gas too. Preference is therefore given to a process where the production of the water-absorbing material herein takes place under inert gas.

It may be advantageous that the absorbent polymeric particle is post-crosslinked, as detailed above. Already post-crosslinked water-absorbing polymeric particles can be coated with the film-forming polymer especially polyurethane. It is likewise possible for the post-crosslinker not to be applied until before heat-treating, i.e., concurrently with the film-forming polymer especially polyurethane in the fluidized bed or after the film-forming polymer-coating step. In the latter version of the process, this can be accomplished, for example, concurrently with the preferred addition of the deagglomerating aid. In all cases, heat-treating is preferably carried out at temperatures in the range from 120 to 160°C.

After the heat-treating step has been concluded, the dried water-absorbing polymeric materials are cooled. To this end, the warm and dry polymer is preferably continuously transferred into a downstream cooler. This can be, for example, a disk cooler, a Nara paddle cooler or a screw cooler.

### Example 1 - Coating of ASAP 510 Z commercial product with Permax 120

The 800 - 850 µm fraction was sieved out of the commercially available product ASAP 510 Z (BASF AG) having the following properties and was then coated with Permax 120 according to the present invention.

ASAP 510 Z (properties before sieving):
CRC = 29.0 g/g
AUL 0.7 psi = 24.5 g/g
SFC = 50 ×10⁻⁷ [cm³s/g]

ASAP 510 Z (properties of the 800 - 850 µm fraction only):
CS-CRC = 32.5 g/g
CS-AUL 0.7 psi = 26.4 g/g
CS-SFC = 66 × 10⁻⁷ [cm³s/g]

A Wurster laboratory coater was used, the amount of absorbent polymer (ASAP 510 Z, 800 - 850 µm in this case) used was 500 g, the Wurster tube was 50 mm in diameter and 150 mm in length, the gap width (distance from base plate) was 15 mm, the Wurster apparatus was conical with a lower diameter of 150 mm expanding to an upper diameter of 300 mm, the carrier gas used was nitrogen having a temperature of 24°C, the gas speed was 3.1 m/s in the Wurster tube and 0.5 m/s in the surrounding annular space.

The polymer dispersion was atomized using a nitrogen-driven two-material nozzle, opening diameter 1.2 mm, the nitrogen temperature being 28°C. The Permax 120 was sprayed from a 41% by weight neat aqueous dispersion whose temperature was 24°C, at a rate of 183 g of dispersion in the course of 65 min. In the process, 15% by weight of Permax was applied to the surface of the absorbent polymer. The amount reported is based on the absorbent polymer used.

Two further runs were carried out in completely the same way except that the add-on level of the Permax was reduced: 5% by weight and 10% by weight.

The coated material was subsequently removed and evenly distributed on Teflonized trays (to avoid sintering together) and dried in a vacuum cabinet at 150°C for 2 hours. Clumps were removed by means of a coarse sieve (1000 µm) and the polymers were characterized as follows:

| Loading with Permax 120 | CS-CRC [g/g] | CS-AUL 0.7 psi [g/g] | CS-SFC [× 10⁻⁷ cm³s/g] |
|---|---|---|---|
| 5% by weight | 27.4 | 23.5 | 764 |
| 10% by weight | 23.1 | 22.0 | 1994 |
| 15% by weight | 21.5 | 20.2 | 2027 |

The properties of these polymers thus coated are accordingly far outside the usual ranges.

### Example 2 - Coating of ASAP 510 Z commercial product with Permax 200

The 800 - 850 µm fraction was sieved out of the commercially available product ASAP 510 Z (BASF AG) having the following properties and was then coated with Permax 200 according to the present invention.

ASAP 510 Z (properties before sieving) as reported in Example 1.

A Wurster laboratory coater was used as in Example 1, the amount of absorbent polymer (ASAP 510 Z, 800 - 850 µm in this case) used was 1000 g, the Wurster tube was 50 mm in diameter and 150 mm in length, the gap width (distance from base plate) was 15 mm, the Wurster apparatus was conical with a lower diameter of 150 mm expanding to an upper diameter of 300 mm, the carrier gas used was nitrogen having a temperature of 24°C, the gas speed was 2.0 m/s in the Wurster tube and 0.5 m/s in the surrounding annular space.

The polymer dispersion was atomized using a nitrogen-driven two-material nozzle, opening diameter 1.2 mm, the nitrogen temperature being 27°C. The Permax 200 was sprayed from a 22% by weight neat aqueous dispersion whose temperature was 24°C, at a rate of 455 g of dispersion in the course of 168 min. In the process, 10% by weight of Permax was applied to the surface of the absorbent polymer. The amount reported is based on the absorbent polymer used.

Three further runs were carried out in completely the same way except that the add-on level of the Permax was reduced: 2.5% by weight, 5.0% by weight and 7.5% by weight.

The coated material was subsequently removed and evenly distributed on Teflonized trays (to avoid sintering together) and dried in a vacuum cabinet at 150°C for 2 hours. Clumps were removed by means of a coarse sieve (1000 µm) and the polymers were characterized as follows:

| Loading with Permax 200 | CS-CRC [g/g] | CS-AUL 0.7 psi [g/g] | CS-SFC [× 10⁻⁷ cm³s/g] |
|---|---|---|---|
| 2.5% by weight | 29.7 | 24.7 | 234 |
| 5.0% by weight | 27.5 | 25.3 | 755 |
| 7.5% by weight | 25.6 | 23.8 | 1082 |
| 10.0% by weight | 23.2 | 24.4 | 1451 |

The properties of these coated polymers are accordingly far outside the usual ranges.

### Example 3: Use of a deagglomerating aid (calcium phosphate) before heat treatment

The run of Example 2 with 10% of Permax 200 was repeated, however, the polymer coated with the dispersion was transferred to a laboratory tumble mixer and 1.0% by weight of tricalcium phosphate type C13-09 (from Budenheim, Mainz) based on polymer was added and mixed dry with the coated polymer for about 10 minutes. Thereafter the polymer was transferred into a laboratory fluidized bed dryer (diameter about 70 mm) preheated to 150°C and, following a residence time of 30 minutes, the following properties were measured:
CS-CRC = 22.2 g/g
CS-AUL = 22.3 g/g
CS-SFC = 1483 × 10⁻⁷ [cm³s/g]

There was no clumping whatsoever during the heat treatment in the fluidized bed, so that the fluidized bed remained very stable and as was demonstrated by subsequent sieving through a 1000 µm sieve.

### Example 4: Use of a deagglomerating aid (Aerosil 90) before heat treatment

The run of Example 2 with 10% of Permax 200 was repeated. However, the polymer coated with the dispersion was transferred to a laboratory tumble mixer and 1.0% by weight Aerosil 90 (from Degussa) based on polymer was added and mixed dry with the coated polymer for about 10 minutes. Thereafter the polymer was placed in a layer of 1.5 - 2.0 cm in an open glass 5 cm in diameter and 3 cm in height and heat treated in a forced-air drying cabinet at 150°C for 120 minutes. The polymer remained completely flowable, and did not undergo any caking or agglomeration.

The following properties were measured:
CS-CRC = 23.6 g/g
CS-AUL = 23.4 g/g
CS-SFC = 1677 × 10⁻⁷ [cm³s/g]

### Example 5:

The same Wurster laboratory coater as in Example 1 was used, the amount of absorbent polymer (ASAP 510 Z, 800-850 µm fraction) used was 1000 g, the Wurster tube was 50 mm in diameter and 150 mm in length, the gap width (distance from base plate) was 15 mm, the Wurster apparatus was conical with a lower diameter of 150 mm expanding to an upper diameter of 300 mm, the carrier gas used was nitrogen having a temperature of 22°C, the gas speed was 2.0 m/s in the Wurster tube and 0.5 m/s in the surrounding annular space.

Estane X-1007-040P was dissolved to yield a 5 wt.% solution in tetrahydrofurane. The polymer solution was atomized using a nitrogen-driven two-material nozzle, opening diameter 1.2 mm, the nitrogen and solution temperature being 22°C. The solution was sprayed at a rate of 586 g of solution in the course of 106 min. In this process, 2.9% by weight of Estane X-1007-040P was applied to the surface of the absorbent polymer. The amount of film-forming polymer Estane X-1007-040P reported is based on the absorbent polymer used.

The coated material was subsequently removed and evenly distributed on teflonized trays (to avoid sintering together) and dried in a vacuum cabinet at 150°C for 2 hours. Clumps were removed by means of a coarse sieve (1000 µm) and the polymer was characterized as follows:

| Loading with Estane X-1007-040P | CS-CRC [g/g] | CS-AUL 0.7 psi [g/g] | CS-SFC [× 10⁻⁷ cm³s/g] |
|---|---|---|---|
| 2.9% by weight | 25.7 | 18.4 | 443 |

### Example 6:

The same Wurster laboratory coater as in Example 1 was used, the amount of absorbent polymer (ASAP 510 Z, 800-850 µm fraction) used was 1000 g, the Wurster tube was not used in this example. The carrier gas used was nitrogen having a temperature of 22°C, and the gas speed was 1.09 - 1.26 m/s.

Estane X-1007-040P was dissolved to yield a 5 wt.% solution in tetrahydrofurane. The polymer solution was atomized using a nitrogen-driven two-material nozzle, opening diameter 1.2 mm, the nitrogen and solution temperature being 23°C. The solution was sprayed at a rate of 500 g of solution in the course of 72 min. In this process, 2.5% by weight of Estane X-1007-040P was applied to the surface of the absorbent polymer. The amount of film-forming polymer Estane X-1007-040P reported is based on the absorbent polymer used.

The coated material was subsequently removed and evenly distributed on teflonized trays (to avoid sintering together) and dried in a vacuum cabinet at 150°C for 2 hours. Clumps were removed by means of a coarse sieve (1000 µm) and the polymer was characterized as follows:

| Loading with Estane X-1007-040P | CS-CRC [g/g] | CS-AUL 0.7 psi [g/g] | CS-SFC [× 10⁻⁷ cm³s/g] |
|---|---|---|---|
| 2.5% by weight | 21.1 | 17.9 | 943 |

The following is the procedure to make the water-absorbing polymer AM0127, as used in the examples below:

Unless stated, all compounds are obtained by Merck, and used w/o purification.

To 2000 g of glacial acrylic acid (AA), an appropriate amount of the core crosslinker (e.g., 1.284 g MethyleneBisAcrylAmide, MBAA, from Aldrich Chemicals) is added and allowed to dissolve at ambient temperature. An amount of water is calculated (6331 g) so that the total weight of all ingredients for the polymerization equals 10000 g (i.e., the concentration of AA is 20 w/w-%). 2000 mg of the initiator ("V50" = 2,2'-azobis (N,N'-dimethylenelsobutyramidine) dihydrochloride, from Waco Chemicals) are dissolved in approx. 40 ml of this calculated amount of the deionized water. 1665.3 g of 50% NaOH are weighted out separately in a Teflon or plastic beaker.

A 16,000 ml resin kettle (equipped with a four-necked glass cover closed with septa, suited for the introduction of a thermometer, syringe needles) is charged with ~ 5 kg ice (prepared from de-ionized water - the amount of this ice is subtracted from the amount of DI water above) Typically, a magnetic stirrer, capable of mixing the whole content (when liquid), is added. The 50% NaOH is added to the ice, and the resulting slurry is stirred. Then, the acrylic acid/MBAA is added within 1 - 2 minutes, while stirring is continued, and the remaining water is added. The resulting solution is clear, all ice melted, and the resulting temperature is typically 15 - 25°C. At this point, the initiator solution is added.

Then, the resin kettle is closed, and a pressure relief is provided, e.g., by puncturing two syringe needles through the septa. The solution is then spurged vigorously with argon via a 60 cm injection needle while stirring at ~ 600 RPM. Stirring is discontinued after ~ 10 minutes, while argon spurging is continued, and two photo lamps ("Twinlite") are placed on either side of the vessel. The solution typically starts to gel after 45 - 60 minutes total. At this point, persistent bubbles form on the surface of the gel, and the argon injection needle is raised above the surface of the gel. Purging with argon is continued at a reduced flow rate. The temperature is monitored; typically it rises from 20°C to 60 - 70°C within 60 - 90 minutes. Once the temperature drops below 60°C, the kettle is transferred into a circulation oven and kept at 60°C for 15 - 18 hours.

After this time, the resin kettle is allowed to cool, and the gel is removed into a flat glass dish. The gel is then broken or cut with scissors into small pieces, and transferred into a vacuum oven, where it is dried at 100°C/ maximum vacuum. Once the gel has reached a constant weight (usually 3 days), it is ground using a mechanical mill (e.g., IKA mill), and sieved to 150 - 850 µm. At this point, various parameters as used herein may be determined.

(This water-absorbing polymer AM0127 had no post-crosslinking.)

### Further examples:

The following are other water-absorbing materials made by the process described above in Example 1, using the conditions and material specified in the table (ASAP 510 being available from BASF):

| Water-absorbing material | Water-absorbing polymer | Particle size (um) | Elastomeric polymer | Conc. Solvent | Coating Level by spraying | Max process temp (°C) | Coat time (min) |
|---|---|---|---|---|---|---|---|
| CP4-P120-15% | ASAP 510Z | 800-850 | Permax 120 | 41% water | 15% | 27.2 | 61.6 |
| CP9-P200-10% | ASAP 510Z | 800-850 | Permax 200 | 22% water | 10% | 29.4 | 81.9 |
| CP14-Xf-8.3% | ASAP 510Z | 800-850 | X-1007-040P | 5% THF | 8.30% | 32.8 | 99 |
| CP16-P200-10% | ASAP 510z | 150-850 | Permax 200 | 22% water | 10% | 28.3 | 86 |
| CP27-P200-15%, 1% tricalcium phosphate | AM0127 | 600-850 | Permax 200 | 22% water | 15% | 30.6 | 105 |

The particle size distribution of the ASAP 510Z bulk material and the sieved fraction of ASAP510Z polymer particles with a particle size of 800-850 microns, 150-850 microns and 600-850 microns, as used above, is as follows:

| ASAP 510Z (bulk distribution) | % | ASAP 510z (800-850 um) | % |
|---|---|---|---|
| < 200 um | 7% | 400 um | 4% |
| 250- 300 um | 18% | 500 um | 11% |
| 350- 400 um | 33% | 600 um | 25% |
| 500 um | 20% | 700 um | 33% |
| 600 um | 12% | 800 um | 25% |
| 700 um | 5% | TOTAL | 98% |
| 800 um | 2% | (mean: 700um) | |
| TOTAL | 97% | | |

| ASAP510 150-850 | % | AM0127 600-850 | % |
|---|---|---|---|
| 150um | 1.7% | <600 | 1.98% |
| 200um | 6.4% | 600um | 4.77% |
| 300um | 11.3% | 700um | 49.11% |
| 400um | 15.5% | 800um | 41.49% |
| 500um | 16.6% | 850um | 2.63% |
| 600um | 15.5% | | |
| 700um | 21.1% | | |
| 800um | 11.9% | | |

The materials obtained by the processes described above were submitted to the QUICs test, 4 hour CCRC test and CS-SFC test described herein and the values below were obtained. Also tested were two prior art materials, referred to as comparison water-absorbing materials.

| | | | SAC" | QUICs | CCRC | CS-SFC | ADI |
|---|---|---|---|---|---|---|---|
| Water-absorbing material of the absorbent structures of the invention: | Annealing conditions | | | | | 10⁻⁷ cm³ sec/g | |
| CP4-P120-15%, ASAP510Z (800-850µm) | 2h 150°C | | 27.204 | 22.6 | 21.89 | 2324.2 | 5.88 |
| CP9-P200-10%, ASAP510Z (8000-850µm) | 2h 150°C | | 30.569 | 24.2 | 23.79 | 1727.2 | 6.63 |
| CP14-Xf-8.3%, ASAP510Z (800-850µm) | 2h 150°C | | 29.122 | 32.0 | 21.60 | 1379.9 | 3.28 |
| CP16-P200-10%, ASAP510Z (150-850µm) | 2h 150°C | | 27.276 | 20.0 | 23.47 | 1356.5 | 4.85 |
| CP27-P200-15%, AM0127 (600-850µm) with the addition of 1% Tricalcium phosphate | 16h | | 63.822 | 77.5 | 34.05 | 276.3 | 9.99 |
| | 150°C/2h | | | | | | |
| | 100°C | | | | | | |
| Comparison water-absorbing materials: | | | | | | | |
| W 52521^{#} | 16h | | 24.278 | 3.5 | 22.95 | 189.0 | 0.6 |
| | 150°C/2h | | | | | | |
| | 100°C | | | | | | |
| AMO 0127 base polymer 150-850um | 16h | | 78.2 | -3.1 | | 0 | |
| | 150°C/2h | | | | | | |
| | 100°C | | | | | | |

| | | | | | | | |
|---|---|---|---|---|---|---|---|
| # W52521: water-absorbing material, containing water-absorbing polymer particles, available from Stockhausen. | | | | | | | |

### Methods used herein:

The measurements should be carried out, unless otherwise stated, at an ambient temperature of 23 ± 2°C and a relative humidity of 50 ± 10%. The water-absorbing polymeric particles are thoroughly mixed through before measurement.

### CRC (Centrifuge Retention Capacity)

This method determines the free swellability of the water-absorbing material or polymer in a teabag. To determine CRC, 0.2000 +/- 0.0050 g of dried polymer or material (particle size fraction 106 - 850 µm or as specifically indicated in the examples which follow) is weighed into a teabag 60 x 85 mm in size, which is subsequently sealed shut. The teabag is placed for 30 minutes in an excess of 0.9% by weight sodium chloride solution (at least 0.831 of sodium chloride solution/1 g of polymer powder). The teabag is subsequently centrifuged at 250 g for 3 minutes. The amount of liquid is determined by weighing the centrifuged teabag. The procedure corresponds to that of EDANA recommended test method No. 441.2-02 (EDANA = European Disposables and Nonwovens Association). The teabag material and also the centrifuge and the evaluation are likewise defined therein.

### CS-CRC (Core Shell Centrifuge Retention Capacity)

CS-CRC is carried out completely analogously to CRC, except that the sample's swelling time is extended from 30 min to 240 min.

### AUL (Absorbency Under Load 0.7 psi)

Absorbency Under Load is determined similarly to the absorption under pressure test method No. 442.2-02 recommended by EDANA (European Disposables and Nonwovens Association), except that for each example the actual sample having the particle size distribution reported in the example is measured.

The measuring cell for determining AUL 0.7 psi is a Plexiglas cylinder 60 mm in internal diameter and 50 mm in height. Adhesively attached to its underside is a stainless steel sieve bottom having a mesh size of 36 µm. The measuring cell further includes a plastic plate having a diameter of 59 mm and a weight which can be placed in the measuring cell together with the plastic plate. The weight of the plastic plate and the weight together weigh 1345 g. AUL 0.7 psi is determined by determining the weight of the empty Plexiglas cylinder and of the plastic plate and recording it as W₀. Then 0.900 +/- 0.005 g of water-absorbing polymer or material (particle size distribution 150 - 800 µm or as specifically reported in the examples which follow) is weighed into the Plexiglas cylinder and distributed very uniformly over the stainless steel sieve bottom. The plastic plate is then carefully placed in the Plexiglas cylinder, the entire unit is weighed and the weight is recorded as Wₐ. The weight is then placed on the plastic plate in the Plexiglas cylinder. A ceramic filter plate 120 mm in diameter, 10 mm in height and 0 in porosity (Duran, from Schott) is then placed in the middle of the Petri dish 200 mm in diameter and 30 mm in height and sufficient 0.9% by weight sodium chloride solution is introduced for the surface of the liquid to be level with the filter plate surface without the surface of the filter plate being wetted. A round filter paper 90 mm in diameter and < 20 µm in pore size (S&S 589 Schwarzband from Schleicher & Schüll) is subsequently placed on the ceramic plate. The Plexiglas cylinder holding the material or polymer is then placed with the plastic plate and weight on top of the filter paper and left there for 60 minutes. At the end of this period, the complete unit is taken out of the Petri dish from the filter paper and then the weight is removed from the Plexiglas cylinder. The Plexiglas cylinder holding swollen water-absorbing material or polymer is weighed out together with the plastic plate and the weight is recorded as W_{b}.

Absorbency under load (AUL) is calculated as follows: $AUL 0.7 psi \left[g/g\right] = \left[{W}_{b}-{W}_{a}\right] / \left[{W}_{a}-{W}_{0}\right]$
AUL 0.3 psi and 0.5 psi are measured similarly at the appropriate lower pressure.
CS-AUL (Core Shell Absorption under load 0.7 psi)

The measuring cell for determining CS-AUL 0.7 psi is a Plexiglas cylinder 60 mm in internal diameter and 50 mm in height. Adhesively attached to its underside is a stainless steel sieve bottom having a mesh size of 36 µm (Steel 1.4401, wire diameter 0.028 mm, from Weisse & Eschrich). The measuring cell further includes a plastic plate having a diameter of 59 mm and a weight which can be placed in the measuring cell together with the plastic plate. The weight of the plastic plate and the weight together weigh 1345 g. AUL 0.7 psi is determined by determining the weight of the empty Plexiglas cylinder and of the plastic plate and recording it as W₀. Then 0.900 +/- 0.005 g of water-absorbing polymer (particle size distribution 150 - 800 µm or as specifically reported in the example which follows) is weighed into the Plexiglas cylinder and distributed very uniformly over the stainless steel sieve bottom. The plastic plate is then carefully placed in the Plexiglas cylinder, the entire unit is weighed and the weight is recorded as Wₐ. The weight is then placed on the plastic plate in the Plexiglas cylinder. A round filter paper with a diameter of 90 mm (No. 597 from Schleicher & Schüll) is placed in the center of a 500 ml crystallizing dish (from Schott) 115 mm in diameter and 65 mm in height. 200 ml of 0.9% by weight sodium chloride solution are then introduced and the Plexiglas cylinder holding the polymer or material is then placed with the plastic plate and weight on top of the filter paper and left there for 240 minutes. At the end of this period, the complete unit is taken out of the Petri dish from the filter paper and adherent liquid is drained off for 5 seconds. Then the weight is removed from the Plexiglas cylinder. The Plexiglas cylinder holding swollen water-absorbing material or polymer is weighed out together with the plastic plate and the weight is recorded as W_{b}.

Absorbency under load (AUL) is calculated as follows: $CS - AUL 0.7 psi \left[g/g\right] = \left[{W}_{b}-{W}_{a}\right] / \left[{W}_{a}-{W}_{0}\right]$
AUL 0.3 psi and 0.5 psi are measured similarly at the appropriate lower pressure.

### Saline Flow Conductivity (SFC)

The method to determine the permeability of a swollen gel layer is the "Saline Flow Conductivity" also known as "Gel Layer Permeability" and is described in EP A 640 330. The equipment used for this method has been modified as described below.

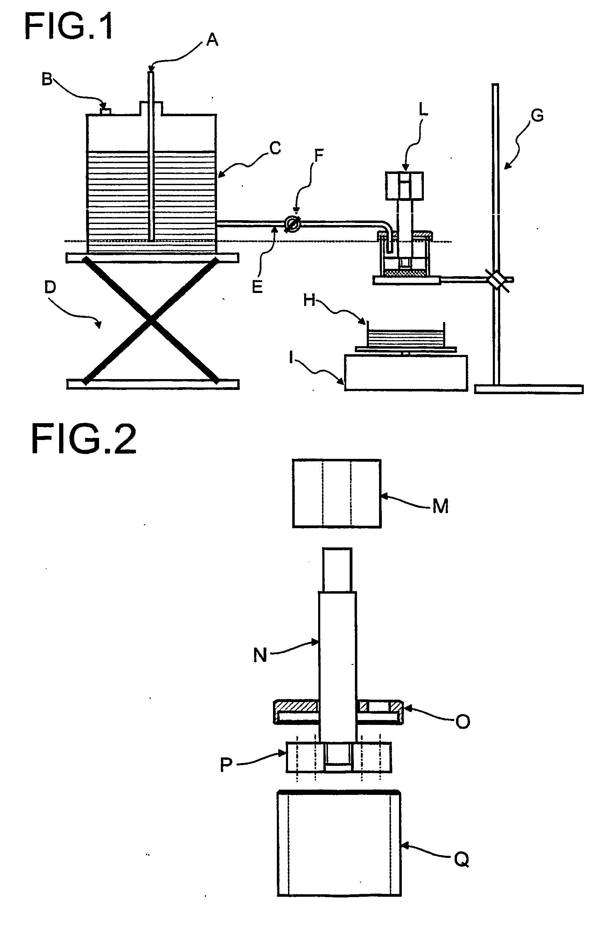

Figure 1 shows the permeability measurement equipment set-up with the open-ended tube for air admittance A, stoppered vent for refilling B, constant hydrostatic head reservoir C, Lab Jack D, delivery tube E, stopcock F, ring stand support G, receiving vessel H, balance I and the SFC apparatus L.
Figure 2 shows the SFC apparatus L consisting of the metal weight M, the plunger shaft N, the lid O, the center plunger P und the cylinder Q.

The cylinder Q has an inner diameter of 6.00 cm (area = 28.27 cm²). The bottom of the cylinder Q is faced with a stainless-steel screen cloth (mesh width: 0.036 mm; wire diameter: 0.028 mm) that is bi-axially stretched to tautness prior to attachment. The plunger consists of a plunger shaft N of 21.15 mm diameter. The upper 26.0 mm having a diameter of 15.8 mm, forming a collar, a perforated center plunger P which is also screened with a stretched stainless-steel screen (mesh width: 0.036 mm; wire diameter: 0.028 mm), and annular stainless steel weights M. The annular stainless steel weights M have a center bore so they can slip on to plunger shaft and rest on the collar. The combined weight of the center plunger P, shaft and stainless-steel weights M must be 596 g (± 6g), which corresponds to 0.30 PSI over the area of the cylinder. The cylinder lid O has an opening in the center for vertically aligning the plunger shaft N and a second opening near the edge for introducing fluid from the reservoir into the cylinder Q.

The cylinder Q specification details are:
Outer diameter of the Cylinder: 70.35 mm
Inner diameter of the Cylinder: 60.0 mm
Height of the Cylinder: 60.5 mm

The cylinder lid O specification details are:
Outer diameter of SFC Lid: 76.05 mm
Inner diameter of SFC Lid: 70.5 mm
Total outer height of SFC Lid: 12.7 mm
Height of SFC Lid without collar: 6.35 mm
Diameter of hole for Plunger shaft positioned in the center: 22.25 mm
Diameter of hole in SFC lid: 12.7 mm
Distance centers of above mentioned two holes: 23.5 mm

The metal weight M specification details are:
Diameter of Plunger shaft for metal weight: 16.0 mm
Diameter of metal weight: 50.0 mm
Height of metal weight: 39.0 cm

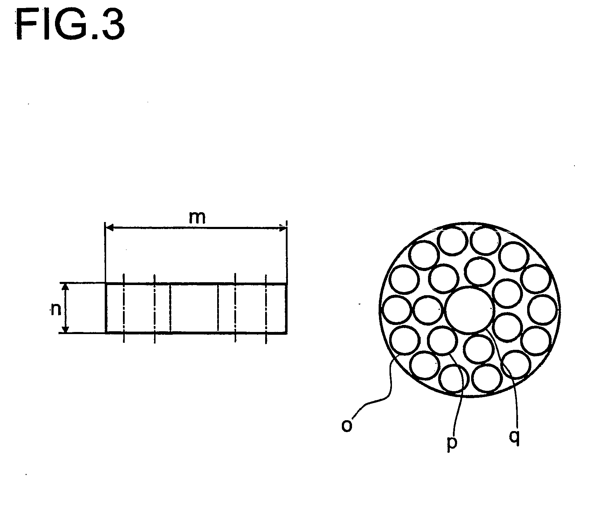

Figure 3 shows the plunger center P specification details:
Diameter m of SFC Plunger center: 59.7 mm
Height n of SFC Plunger center: 16.5 mm
14 holes o with 9.65 mm diameter equally spaced on a 47.8 mm bolt circle and
7 holes p with a diameter of 9.65 mm equally spaced on a 26.7 mm bolt circle 5/8 inches thread q

Prior to use, the stainless steel screens of SFC apparatus, should be accurately inspected for clogging, holes or over stretching and replaced when necessary. An SFC apparatus with damaged screen can deliver erroneous SFC results, and must not be used until the screen has been fully replaced.

Measure and clearly mark, with a permanent fine marker, the cylinder at a height of 5.00 cm (± 0.05 cm) above the screen attached to the bottom of the cylinder. This marks the fluid level to be maintained during the analysis. Maintenance of correct and constant fluid level (hydrostatic pressure) is critical for measurement accuracy.

A constant hydrostatic head reservoir C is used to deliver NaCl solution to the cylinder and maintain the level of solution at a height of 5.0 cm above the screen attached to the bottom of the cylinder. The bottom end of the reservoir air-intake tube A is positioned so as to maintain the fluid level in the cylinder at the required 5.0 cm height during the measurement, i.e., the height of the bottom of the air tube A from the bench top is the same as the height from the bench top of the 5.0 cm mark on the cylinder as it sits on the support screen above the receiving vessel. Proper height alignment of the air intake tube A and the 5.0 cm fluid height mark on the cylinder is critical to the analysis. A suitable reservoir consists of a jar containing: a horizontally oriented L-shaped delivery tube E for fluid delivering, an open-ended vertical tube A for admitting air at a fixed height within the reservoir, and a stoppered vent B for re-filling the reservoir. The delivery tube E, positioned near the bottom of the reservoir C, contains a stopcock F for starting/stopping the delivery of fluid. The outlet of the tube is dimensioned to be inserted through the opening in the cylinder lid O, with its end positioned below the surface of the fluid in the cylinder (after the 5 cm height is attained). The air-intake tube is held in place with an o-ring collar. The reservoir can be positioned on a laboratory jack D in order to adjust its height relative to that of the cylinder. The components of the reservoir are sized so as to rapidly fill the cylinder to the required height (i.e., hydrostatic head) and maintain this height for the duration of the measurement. The reservoir must be capable to deliver liquid at a flow rate of minimum 3 g/sec for at least 10 minutes.

Position the plunger/cylinder apparatus on a ring stand with a 16 mesh rigid stainless steel support screen (or equivalent). This support screen is sufficiently permeable so as to not impede fluid flow and rigid enough to support the stainless steel mesh cloth preventing stretching. The support screen should be flat and level to avoid tilting the cylinder apparatus during the test. Collect the fluid passing through the screen in a collection reservoir, positioned below (but not supporting) the support screen. The collection reservoir is positioned on a balance accurate to at least 0.01 g. The digital output of the balance is connected to a computerized data acquisition system.

### Preparation of reagents

Following preparations are referred to a standard 1 liter volume. For preparation multiple than 1 liter, all the ingredients must be calculated as appropriate.

### Jayco Synthetic Urine

Fill a 1L volumetric flask with de-ionized water to 80% of its volume, add a stir bar and put it on a stirring plate. Separately, using a weighing paper or beaker weigh (accurate to ± 0.01 g) the amounts of the following dry ingredients using the analytical balance and add them into the volumetric flask in the same order as listed below. Mix until all the solids are dissolved then remove the stir bar and dilute to 1L volume with distilled water. Add a stir bar again and mix on a stirring plate for a few minutes more. The conductivity of the prepared solution must be 7.6 ± 0.23 mS/cm.

### Chemical Formula Anhydrous Hydrated

Potassium Chloride (KCl) 2.00 g
Sodium Sulfate (Na2SO4) 2.00 g
Ammonium dihydrogen phosphate (NH4H2PO4) 0.85 g
Ammonium phosphate, dibasic ((NH4)2HPO4) 0.15 g
Calcium Chloride (CaCl2) 0.19 g (2 H2O) 0.25 g
Magnesium chloride (MgCl2) 0.23 g (6 H2O) 0.50 g

To make the preparation faster, wait until total dissolution of each salt before adding the next one. Jayco may be stored in a clean glass container for 2 weeks. Do not use if solution becomes cloudy. Shelf life in a clean plastic container is 10 days.

### 0.118 M Sodium Chloride (NaCl) Solution

Using a weighing paper or beaker weigh (accurate to ± 0.01 g) 6.90 g of sodium chloride into a 1L volumetric flask and fill to volume with de-ionized water. Add a stir bar and mix on a stirring plate until all the solids are dissolved. The conductivity of the prepared solution must be 12.50 ± 0.38 mS/cm.

### Test preparation

Using a reference metal cylinder (40 mm diameter; 140 mm height) set the caliper gauge (e.g., Mitotoyo Digimatic Height Gage) to read zero. This operation is conveniently performed on a smooth and level bench top. Position the SFC apparatus without water-absorbing material or polymer under the caliper gauge and record the caliper as L1 to the nearest of 0.01 mm.

Fill the constant hydrostatic head reservoir with the 0.118 M NaCl solution. Position the bottom of the reservoir air-intake tube A so as to maintain the top part of the liquid meniscus in the SFC cylinder at the required 5.0 cm height during the measurement. Proper height alignment of the air-intake tube A at the 5 cm fluid height mark on the cylinder is critical to the analysis.

Saturate an 8 cm fritted disc (7 mm thick; e.g., Chemglass Inc. # CG 201- 51, coarse porosity) by adding excess synthetic urine on the top of the disc. Repeating until the disc is saturated. Place the saturated fritted disc in the hydrating dish and add the synthetic urine until it reaches the level of the disc. The fluid height must not exceed the height of the disc.

Place the collection reservoir on the balance and connect the digital output of the balance to a computerized data acquisition system. Position the ring stand with a 16 mesh rigid stainless steel support screen above the collection dish. This 16 mesh screen should be sufficiently rigid to support the SFC apparatus during the measurement. The support screen must be flat and level.

### Sampling

Samples (of the water-absorbing material or polymer) should be stored in a closed bottle and kept in a constant, low humidity environment. Mix the sample to evenly distribute particle sizes. Remove a representative sample of material to be tested from the center of the container using the spatula. The use of a sample divider is recommended to increase the homogeneity of the sample particle size distribution.

### SFC procedure

Position the weighing funnel on the analytical balance plate and zero the balance. Using a spatula weigh 0.9 g (± 0.05g) of the sample into the weighing funnel. Position the SFC cylinder on the bench, take the weighing funnel and gently, tapping with finger, transfer the sample into the cylinder being sure to have an evenly dispersion of it on the screen. During the sample transfer, gradually rotate the cylinder to facilitate the dispersion and get homogeneous distribution. It is important to have an even distribution of particles on the screen to obtain the highest precision result. At the end of the distribution the sample material must not adhere to the cylinder walls. Insert the plunger shaft into the lid central hole then insert the plunger center into the cylinder for few centimeters. Keeping the plunger center away from the sample insert the lid in the cylinder and carefully rotate it until the alignment between the two is reached. Carefully rotate the plunger to reach the alignment with lid then move it down allowing it to rest on top of the dry sample. Insert the stainless steel weight to the plunger rod and check if the lid moves freely. Proper seating of the lid prevents binding and assures an even distribution of the weight on the gel bed.

The thin screen on the cylinder bottom is easily stretched. To prevent stretching, apply a sideways pressure on the plunger rod, just above the lid, with the index finger while grasping the cylinder portion of the apparatus. This "locks" the plunger in place against the inside of the cylinder so that the apparatus can be lifted. Place the entire apparatus on the fritted disc in the hydrating dish. The fluid level in the dish should not exceed the height of the fritted disc. Care should be taken so that the layer does not loose fluid or take in air during this procedure. The fluid available in the dish should be enough for all the swelling phase. If needed, add more fluid to the dish during the hydration period to ensure there is sufficient synthetic urine available. After a period of 60 minutes, place the SFC apparatus under the caliper gauge and record the caliper as L2 to the nearest of 0.01 mm. Calculate, by difference L2 - L1, the thickness of the gel layer as L0 to the nearest ± 0.1 mm. If the reading changes with time, record only the initial value.

Transfer the SFC apparatus to the support screen above the collection dish. Be sure, when lifting the apparatus, to lock the plunger in place against the inside of the cylinder. Position the constant hydrostatic head reservoir such that the delivery tube is placed through the hole in the cylinder lid. Initiate the measurement in the following sequence:
a) Open the stopcock of the constant hydrostatic head reservoir and permit the fluid to reach the 5 cm mark. This fluid level should be obtained within 10 seconds of opening the stopcock.
b) Once 5 cm of fluid is attained, immediately initiate the data collection program.

With the aid of a computer attached to the balance, record the quantity of fluid passing through the gel layer versus time at intervals of 20 seconds for a time period of 10 minutes. At the end of 10 minutes, close the stopcock on the reservoir. The data from 60 seconds to the end of the experiment are used in the calculation. The data collected prior to 60 seconds are not included in the calculation. Perform the test in triplicate for each sample material.

Evaluation of the measurement remains unchanged from EP-A 640 330. Through-flux is captured automatically.

Saline flow conductivity (SFC) is calculated as follows: $SFC \left[{cm}^{3}⁢s/g\right] = \left(Fg\left(t=0\right) x {L}_{0}\right) / \left(d x A x WP\right) ,$
where Fg(t=0) is the through-flux of NaCl solution in g/s, which is obtained from a linear regression analysis of the Fg(t) data of the through-flux determinations by extrapolation to t=0, L₀ is the thickness of the gel layer in cm, d is the density of the NaCl solution in g/cm³, A is the area of the gel layer in cm² and WP is the hydrostatic pressure above the gel layer in dyn/cm².

### CS-SFC (Core Shell Saline Flow Conductivity)

CS-SFC is determined completely analogously to SFC, with the following changes:

To modify the SFC the person skilled in the art will design the feed line including the stopcock in such a way that the hydrodynamic resistance of the feed line is so low that prior to the start of the measurement time actually used for the evaluation an identical hydrodynamic pressure as in the SFC (5 cm) is attained and is also kept constant over the duration of the measurement time used for the evaluation.
- the weight of the sample (of the water-absorbing material or polymer) used is 1.50 +/- 0.05 g
- a 0.9% by weight sodium chloride solution is used as solution to preswell the sample and for through-flux measurement
- the preswell time of the sample for measurement is 240 minutes
- for preswelling, a filter paper 90 mm in diameter (Schleicher & Schüll, No 597) is placed in a 500 ml crystallizing dish (Schott, diameter = 115 mm, height = 65 mm) and 250 ml of 0.9% by weight sodium chloride solution are added, then the SFC measuring cell with the sample is placed on the filter paper and swelling is allowed for 240 minutes
- the through-flux data are recorded every 5 seconds, for a total of 3 minutes
- the points measured between 10 seconds and 180 seconds are used for evaluation and Fg(t=0) is the through-flux of NaCl solution in g/s which is obtained from a linear regression analysis of the Fg(t) data of the through-flux determinations by extrapolation to t=0
- the stock reservoir bottle in the SFC-measuring apparatus for through-flux solution contains about 5 kg of sodium chloride solution.

### Cylinder Centrifuge Retention Capacity (4 hours CCRC)

The Cylinder Centrifuge Retention Capacity (CCRC) method determines the fluid retention capacity of the water-absorbing materials or polymers (sample) after centrifugation at an acceleration of 250g, herein referred to as absorbent capacity. Prior to centrifugation, the sample is allowed to swell in excess saline solution in a rigid sample cylinder with mesh bottom and an open top.

Duplicate sample specimens are evaluated for each material tested and the average value is reported.

The CCRC can be measured at ambient conditions by placing the sample material (1.0 +/- 0.001 g) into a pre-weighed (+/-- 0.01 g) Plexiglas sample container that is open at the top and closed on the bottom with a stainless steel mesh (400) that readily allows for saline flow into the cylinder but contains the absorbent particles being evaluated. The sample cylinder approximates a rectangular prism with rounded-edges in the 67 mm height dimension. The base dimensions (78 X 58 mm OD, 67.2 X 47.2 MM ID) precisely match those of modular tube adapters, herein referred to as the cylinder stand, which fit into the rectangular rotor buckets (Heraeus # 75002252, VWR # 20300-084) of the centrifuge (Heraeus Megafuge 1.0; Heraeus # 75003491, VWR # 20300-016).

The loaded sample cylinders are gently shaken to evenly distribute the sample across the mesh surface and then placed upright in a pan containing saline solution. The cylinders should be positioned to ensure free flow of saline through the mesh bottom. Cylinders should not be placed against each other or against the wall of the pan, or sealed against the pan bottom. The sample is allowed to swell, without confining pressure and in excess saline, for 4 hours.

After 4 hours, the cylinders are immediately removed from the solution. Each cylinder is placed (mesh side down) onto a cylinder stand and the resulting assembly is loaded into the rotor basket such that the two sample assemblies are in balancing positions in the centrifuge rotor.

The samples are centrifuged for 3 minutes (± 10s) after achieving the rotor velocity required to generate a centrifugal acceleration of 250±5g at the bottom of the cylinder stand. The openings in the cylinder stands allow any solution expelled from the absorbent by the applied centrifugal forces to flow from the sample to the bottom of the rotor bucket where it is contained. The sample cylinders are promptly removed after the rotor comes to rest and weighed to the nearest 0.01 g.

The cylinder centrifuge retention capacity expressed as grams of saline solution absorbed per gram of sample material is calculated for each replicate as follows: $CCRC = \frac{{m}_{CS} - \left({m}_{Cb}+{m}_{S}\right)}{{m}_{S}} \left[\frac{g}{g}\right]$
where:
- m_{CS}:: is the mass of the cylinder with sample after centrifugation [g]
- m_{Cb}:: is the mass of the dry cylinder without sample [g]
- m_{S}:: is the mass of the sample without saline solution [g]
The CCRC referred to herein is the average of the duplicate samples reported to the nearest 0.01 g/g.

### Particle size distribution

Particle size distribution is determined by the EDANA (European Disposables and Nonwovens Association) recommended test method No. 420.2-02 "Particle Size Distribution".

### 16h extractables

The level of extractable constituents in the water-absorbing polymeric particles is determined by the EDANA (European Disposables and Nonwovens Association) recommended test method No. 470.2-02 "Determination of extractable polymer content by potentiometric titration". Extraction time is 16 hours.

### pH value

The pH of the water-absorbing polymeric particles is determined by the EDANA (European Disposables and Nonwovens Association) recommended test method No. 400.2-02 "Determination of pH".

### Surface tension of aqueous extract

0.50 g of the water-absorbing polymeric particles is weighed into a small glass beaker and admixed with 40 ml of 0.9% by weight salt solution. The contents of the beaker are magnetically stirred at 500 rpm for 3 minutes and then allowed to settle for 2 minutes. Finally, the surface tension of the supernatant aqueous phase is measured with a K10-ST digital tensiometer or a comparable apparatus having a platinum plate (from Kruess). The measurement is carried out at a temperature of 23°C.

### Moisture content of base polymer

The water content of the water-absorbing polymeric particles is determined by the EDANA (European Disposables and Nonwovens Association) recommended test method No. 430.2-02 "Moisture content".

### Quality Index for Core Shells (QUICS): method to calculate the QUICS value (QUICS method):

The water-absorbing material herein is such that it allows effective absorption of fluids, whilst providing at the same time a very good permeability of the water-absorbing material, once it has absorbed the fluids and once it is swollen, as, for example, may be expressed in CS-SFC value, described herein.

The inventors found that the change of the absorbent capacity of water-absorbing material when it is submitted to grinding, is a measure to determine whether the water-absorbing material exerts a pressure, which is high enough to ensure a much improved permeability of the water-absorbing material (when swollen) of the absorbent structures of the invention, providing ultimately an improved performance in use.

The water-absorbing material comprises particles with a core-shell structure described herein, whereby the shell of elastomeric polymers exerts said significant pressure onto said core of water-absorbing polymers (whilst still allowing high quantities of fluid to be absorbed). The inventors have found that without such a shell, the water-absorbing material may have a good fluid absorbent capacity, but it will have a very poor permeability, in comparison to the water-absorbing material of the absorbent structures of the invention. Thus, the inventors have found that this internal pressure that is generated by the shell is beneficial for the ultimate performance of water-absorbing material herein.

Then, the change of the absorbent capacity of the water-absorbing material, when the particles thereof are broken, e.g., when the shell on the particles (e.g., of the water-absorbing polymers) is removed or destroyed, is a measure to determine whether the water-absorbing material comprises particles with a shell that exerts a pressure onto the core, which is high enough to ensure a much improved permeability of the water-absorbing material (when swollen) herein.

The following is the method used herein to determine the absorbent capacity of the water-absorbing material, and the absorbent capacity of the same water-absorbing material after submission to the grinding method (e.g., to destroy the shells), to subsequently determine the change of absorbent capacity, expressed as QUICS value.

As absorption fluid, a 0.9% NaCl solution in de-ionized water is used ('saline').

Each initial sample is 70 mg +/- 0.05 mg water-absorbing material of the absorbent structures of the invention ('sample').

Duplicate sample specimens are evaluated for each material tested and the average value is used herein.

### a. Determination of the Saline Absorbent Capacity (SAC) of the waster-absorbing material sample

At ambient temperature and humidity (i.e., 20°C and 50% +/- 10% humidity) and at ambient pressure, the sample is placed into a pre-weighed (+/-- 0.01 g) Plexiglas sample container (QUICS-pot) that is open at the top and closed on the bottom with a stainless steel mesh (400) that readily allows for saline flow into the cylinder but contains the absorbent particles being evaluated. The sample cylinder approximates a rectangular prism with rounded-edges in the 67 mm height dimension. The base dimensions (78 X 58 mm OD, 67.2 X 47.2 MM ID) precisely match those of modular tube adapters, herein referred to as the cylinder stand, which fit into the rectangular rotor buckets (Heraeus # 75002252, VWR # 20300-084) of the centrifuge (Heraeus Megafuge 1.0; Heraeus # 75003491, VWR # 20300-016).

The cylinder with sample is gently shaken to evenly distribute the sample across the mesh surface and it is then placed upright in a pan containing saline solution. A second cylinder with a second sample is prepared in the same manner. The cylinders should be positioned such that to free flow of saline through the mesh bottom is ensured at all times. The cylinders should not be placed against each other or against the wall of the pan, or sealed against the pan bottom. Each sample is allowed to swell, at the ambient conditions above, without confining pressure, for 4 hours. The saline level inside the cylinders is at least 3 cm from the bottom mesh. Optionally, a small amount of a dye may be added to stain the (elastic) shell, e.g., 10 PPM Toluidine Blue, or 10 PPM Chicago Sky Blue 6B.

After 4 hours (+/- 2 minutes), the cylinders are removed from the saline solution. Each cylinder is placed (mesh side down) onto a cylinder stand and the resulting assembly is loaded into the rotor basket of the centrifuge, such that the two sample assemblies are in balancing positions in the centrifuge rotor.

The samples are centrifuged for 3 minutes (± 10s) after achieving the rotor velocity required to generate a centrifugal acceleration of 250±5g at the bottom of the cylinder stand. The openings in the cylinder stands allow any solution expelled from the absorbent by the applied centrifugal forces to flow from the sample to the bottom of the rotor bucket where it is contained. The sample cylinders are promptly removed after the rotor comes to rest and weighed to the nearest 0.01 g.

The Saline Absorbent Capacity (SAC) expressed as grams of saline solution absorbed per gram of sample material is calculated for each replicate as follows: $SAC = \frac{{m}_{CS} - \left({m}_{Cb}+{m}_{S}\right)}{{m}_{S}} \left[\frac{g}{g}\right]$
where:
- m_{CS}:: is the mass of the cylinder with sample after centrifugation [g]
- m_{Cb}:: is the mass of the dry cylinder without sample [g]
- m_{S}:: is the mass of the sample without saline solution [g]

The SAC referred to herein is the average of the duplicate samples reported to the nearest 0.01 g/g.

### b. Grinding of the sample:

After the weight measurements above, the swollen sample obtained above is transferred (under the same temperature, humidity and pressure conditions as set out above) to the centre of a flat Teflon sheet (20 * 20 cm * 1.0 mm) by means of a spatula. The Teflon sheet is supported on a hard, smooth surface, e.g., a standard laboratory bench. The QUICS-pot is weighed back to ensure that a > 95% transfer of the swollen sample to the Teflon sheet has been achieved.

A round glass plate (15 cm diameter, 8 mm thickness) is placed on top of the sample and the sample is thus squeezed between this top glass plate and the bottom support. Two 10 lb. weights are placed on the top glass plate; the top glass plate is rotated twice against the stationary Teflon sheet. (For example, when the water-absorbing material comprises particles with shells, this operation will break or destroy the shell of the swollen particles of the swollen sample, and thus a (swollen) sample of broken particles, or typically particles with a broken or destroyed shell, are obtained.

### c. Determination of the SAC" of the ground (swollen) sample obtained in 2. above:

The ground (swollen) sample obtained above in b) is quantitatively transferred back into the respective QUICS-pot, e.g., with the help of 0.9% NaCl solution from a squirt bottle, so that it is placed in the pot as described above. Each pot of each sample is placed in 0.9% NaCl solution under the same conditions and manner as above, but for 2 hours rather than 4 hours, and the second SAC" of the sample is determined by the centrifugation described above.

N.B.: The time elapsed between the end of the first centrifugation to determine the SAC (in step a.) and the beginning of the step c. to determine the SAC", (i.e., the start of transfer to QUICS pot), should not exceed more than 30 minutes.

### d. QUICS calculation:

Then the QUICS as used herein is determined as follows: $QUICS = 100 * \left(SACʺ\right) / \left(SAC\right) - 100$

### Methods for analyzing the coating polymers or coatings:

### Preparation of films of the elastic film-forming polymer

In order to subject the elastic film-forming polymer used herein to some of the test methods below, including the Wet-elongation test, films need to be obtained of said polymers thereof.

The preferred average (as set out below) caliper of the (dry) films for evaluation in the test methods herein is around 60 µm.

Methods to prepare films are generally known to those skilled in the art and typically comprise solvent casting, hotmelt extrusion or melt blowing films. Films prepared by these methods may have a machine direction that is defined as the direction in which the film is drawn or pulled. The direction perpendicular to the machine direction is defined as the cross-direction.

For the purpose of the invention, the films used in the test methods below are formed by solvent casting, except when the elastic film-forming polymer cannot be made into a solution or dispersion of any of the solvents listed below, and then the films are made by hotmelt extrusion as described below. (The latter is the case when particulate matter from the elastic film-forming polymer is still visible in the mixture of the material or coating agent and the solvent, after attempting to dissolve or disperse it at room temperature for a period between 2 to 48 hours, or when the viscosity of the solution or dispersion is too high to allow film casting.)

The resulting film should have a smooth surface and be free of visible defects such as air bubbles or cracks.

An example to prepare a solvent cast film herein from an elastic film-forming polymer:

The film to be subjected to the tests herein can be prepared by casting a film from a solution or dispersion of said material or coating agent as follows:

The solution or dispersion is prepared by dissolving or dispersing the elastic film-forming polymer, at 10 weight%, in water, or if this is not possible, in THF (tetrahydrofuran), or if this is not possible, in dimethylformamide (DMF), or if this is not possible in methyl ethyl ketone (MEK), or if this is not possible, in dichloromethane or if this is not possible in toluene, or if this is not possible in cyclohexane (and if this is not possible, the hotmelt extrusion process below is used to form a film). Next, the dispersion or solution is poured into a Teflon dish and is covered with aluminum foil to slow evaporation, and the solvent or dispersant is slowly evaporated at a temperature above the minimum film forming temperature of the polymer, typically about 25°C, for a long period of time, e.g., during at least 48 hours, or even up to 7 days. Then, the films are placed in a vacuum oven for 6 hours, at 25°C, to ensure any remaining solvent is removed.

The process to form a film from an aqueous dispersion is as follows:

The dispersion may be used as received from the supplier, or diluted with water as long as the viscosity remains high enough to draw a film (200 - 500 cps). The dispersion solution (5 - 10 ml) is placed onto a piece of aluminum foil that is attached to the stage of the draw down table. The polymer dispersion is drawn using a Gardner metering rod #30 or #60 to draw a film that is 50-100 microns thick after drying. The dispersant is slowly evaporated at a temperature above the minimum film forming temperature of the polymer, typically about 25°C, for a long period of time, e.g., during at least 48 hours, or even up to 7 days. The film is heated in a vacuum oven at 150 °C for a minimum of 5 minutes up to 2h, then the film is removed from the foil substrate by soaking in warm water bath for 5 to 10 min to remove the films from the substrate. The removed film is then placed onto a Teflon sheet and dried under ambient conditions for 24h. The dried films are then sealed in a plastic bag until testing can be performed.

The process to prepare a hotmelt extruded film herein is as follows:

If the solvent casting method is not possible, films of the elastic film-forming polymer 1 herein may be extruded from a hot melt using a rotating single screw extrusion set of equipment operating at temperatures sufficiently high to allow the elastic film-forming polymer to flow. If the polymer has a melting temperature Tm, then the extrusion should take place at least 20 K above said Tm. If the polymer is amorphous (i.e., does not have a Tm), steady shear viscometry can be performed to determine the order to disorder transition for the polymer, or the temperature where the viscosity drops dramatically. The direction that the film is drawn from the extruder is defined as the machine direction and the direction perpendicular to the drawing direction is defined as the cross direction.

| For example | Wet-extensible material | Die Temperature | Screw rpm |
|---|---|---|---|
| 20 | Irogran VP 654/5 | 180 °C | 40 |
| 21 | Elastollan LP 9109 | 170 °C | 30 |
| 22 | Estane 58245 | 180 °C | 30 |
| 23 | Estane 4988 | 180 °C | 30 |
| 24 | Pellethane 2103-70A | 185 °C | 30 |

### Heat-treating of the films:

The heat-treating of the films should, for the purpose of the test methods below, be done by placing the film in a vacuum oven at a temperature which is about 20 K above the highest Tg of the used elastic film-forming polymer, and this is done for 2 hours in a vacuum oven at less than 0.1 Torr, provided that when the elastic film-forming polymer has a melting temperature Tm, the heat-treating temperature is at least 20 K below the Tm, and then preferably (as close to) 20 K above the highest Tg. When the Tg is reached, the temperature should be increased slowly above the highest Tg to avoid gaseous discharge that may lead to bubbles in the film. For example, a material with a hard segment Tg of 70°C might be heat-treated at 90°C for 10 min, followed by incremental increases in temperature until the heat-treating temperature is reached.

If the elastic film-forming polymer has a Tm, then said heat-treating of the films (prepared as set out above and to be tested by the methods below) is done at a temperature which is above the (highest) Tg and at least 20 K below the Tm and (as close to) 20 K above the (highest) Tg. For example, a wet-extensible material that has a Tm of 135°C and a highest Tg (of the hard segment) of 100°C, would be heat-treated at 115°C.

In the absence of a measurable Tg or Tm, the temperature for heat-treating in this method is the same as used in the process for making water-absorbing material. Removal of films, if applicable:

If the dried and optionally heat-treated films are difficult to remove from the film-forming substrate, then they may be placed in a warm water bath for 30 s to 5 min to remove the films from the substrate. The film is then subsequently dried for 6 - 24h at 25°C.

### Wet-elongation Test and Wet-tensile-stress Test:

This test method is used to measure the wet-elongation at break (= extensibility at break) and tensile properties of films of elastic film-forming polymers as used herein, by applying a uniaxial strain to a flat sample and measuring the force that is required to elongate the sample. The film samples are herein strained in the cross-direction, when applicable.

A preferred piece of equipment to do the tests is a tensile tester such as an MTS Synergie100 or an MTS Alliance available from MTS Systems Corporation 14000 Technology Drive, Eden Prairie, MN, USA, with a 25N or 50N load cell. This measures the Constant Rate of Extension in which the pulling grip moves at a uniform rate and the force measuring mechanisms moves a negligible distance (less than 0.13mm) with increasing force. The load cell is selected such that the measured loads (e.g., force) of the tested samples will be between 10 and 90% of the capacity of the load cell.

Each sample is die-cut from a film, each sample being 1 x 1 inch (2.5 x 2.5 cm), as defined above, using an anvil hydraulic press die to cut the film into sample(s) (Thus, when the film is made by a process that does not introduce any orientation, the film may be tested in either direction.). Test specimens (minimum of three) are chosen which are substantially free of visible defects such as air bubbles, holes, inclusions, and cuts. They must also have sharp and substantially defect-free edges.

The thickness of each dry specimen is measured to an accuracy of 0.001 mm with a low pressure caliper gauge such as a Mitutoyo Caliper Gauge using a pressure of about 0.1 psi. Three different areas of the sample are measured and the average caliper is determined. The dry weight of each specimen is measured using a standard analytical balance to an accuracy of 0.001 g and recorded. Dry specimens are tested without further preparation for the determination of dry-elongation, dry-secant modulus, and dry-tensile stress values used herein.

For wet testing, preweighed dry film specimens are immersed in saline solution [0.9% (w/w) NaCl] for a period of 24 hours at ambient temperature (23 +/- 2°C). Films are secured in the bath with a 120-mesh corrosion-resistant metal screen that prevents the sample from rolling up and sticking to itself. The film is removed from the bath and blotted dry with an absorbent tissue such as a Bounty^{©} towel to remove excess or non-absorbed solution from the surface. The wet caliper is determined as noted for the dry samples. Wet specimens are used for tensile testing without further preparation. Testing should be completed within 5 minutes after preparation is completed. Wet specimens are evaluated to determine wet-elongation, wet-secant modulus, and wet-tensile stress.

Tensile testing is performed on a constant rate of extension tensile tester with computer interface such as an MTS Alliance tensile tester with Testworks 4 software. Load cells are selected such that measured forces fall within 10-90% of the cell capacity. Pneumatic jaws, fitted with flat 1 "-square rubber-faced grips, are set to give a gauge length of 1 inch. The specimen is loaded with sufficient tension to eliminate observable slack, but less than 0.05N. The specimens are extended at a constant crosshead speed of 10"/min until the specimen completely breaks. If the specimen breaks at the grip interface or slippage within the grips is detected, then the data is disregarded and the test is repeated with a new specimen and the grip pressure is appropriately adjusted. Samples are run in triplicate to account for film variability.

The resulting tensile force-displacement data are converted to stress-strain curves using the initial sample dimensions from which the elongation, tensile stress, and modulus that are used herein are derived. Tensile stress at break is defined as the maximum stress measured as a specimen is taken to break, and is reported in MPa. The secant modulus at 400% elongation is defined as the slope of the line that intersects the stress-strain curve at 0% and 400% strain. Three stress-strain curves are generated for each elastomeric film coating that is evaluated. The modulus and tensile stress at break, used herein, are the average of the respective values derived from each curve.

### Glass Transition Temperatures

Glass Transition Temperatures (Tg's) are determined for the purpose of this invention by differential scanning calorimetry (DSC). The calorimeter should be capable of heating/cooling rates of at least 20°C/min over a temperature range, which includes the expected Tg's of the sample that is to be tested, e.g., of from -90°C to 250°C, and the calorimeter should have a sensitivity of about 0.2 µW. TA Instruments Q1000 DSC is well-suited to determining the Tg's referred to herein. The material of interest can be analyzed using a temperature program such as: equilibrate at -90°C, ramp at 20°C/min to 120°C, hold isothermal for 5 minutes, ramp 20°C/min to -90°C, hold isothermal for 5 minutes, ramp 20°C/min to 250°C. The data (heat flow versus temperature) from the second heat cycle is used to calculate the Tg via a standard half extrapolated heat capacity temperature algorithm. Typically, 3-5 g of a sample material is weighed (+/- 0.1 g) into an aluminum DSC pan with crimped lid.

As used herein Tg₁ will be a lower temperature than Tg₂.

### Pulsed NMR method to determine weight percentage of the coating or shell

The following describes the method, which can be used to determine the weight percentage of the coating (by weight of the sample of the water-absorbing material) of the water-absorbing particles of said material, whereby said shell comprises elastomeric polymers with (at least one) Tg of less than 60°C, using known Pulsed Nuclear Magnetic Resonance techniques, whereby the size of each spin-echo signal from identical protons (bonded to the molecules of said elastomeric polymer present in a sample) is a measure of the amount of said protons present in the sample and hence a measure of the amount of said molecules of said elastomeric polymer present (and thus the weight percentage thereof - see below) present in the sample.

For the pulsed NMR measurement a Maran 23 Pulsed NMR Analyzer with 26 mm Probe, Universal Systems, Solon, OH, may be used.

The sample will be a water-absorbing material, of which its chemical composition is know, and of which the weight percentage of the coating is to be determined.

To generate a calibration curve for needed for this measurement, water-absorbing materials of the same chemical composition, but with known coating/shell weight percentage levels are prepared as follows: 0% (no coating), 1%, 2%, 3%, 4%, 6%, 8% and 10% by weight. These are herein referred to as 'standards'.

Each standard and the sample must be vacuum dried for 24h at 120°C before the start of a measurement.

For each measurement, 5 grams (with an accuracy of 0.0001 g) of a standard or of a sample is weighed in a NMR tube (for example, Glass sample tubes, 26 mm diameter, at least 15 cm in height).

The sample and the eight standards are placed in a mineral oil dry bath for 45 minutes prior to testing, said dry bath being set at 60°C +/- 1 °C. (The bath temperature is verified by placing a glass tube containing two inches of mineral oil and a thermometer into the dry bath.) For example, a Fisher Isotemp. Dry Bath Model 145, 120V, 50/60 HZ, Cat. #11-715-100, or equivalent can be used.

The standards and the sample should not remain in the dry bath for more than 1 hour prior to testing. The sample and the standards must be analyzed within 1 minute after transfer from the bath to the NMR instrument.

For the NMR measurements, the NMR and RI Multiquant programs of the NMR equipment are started and the measurements are made following normal procedures (and using the exact coating amount [g] for each standard in the computer calculations). The centre of the spin echo data is used when analyzing the data, using normal procedures.

Then, the sample, prepared as above, is analyzed in the same manner and using the computer generated data regarding the standards, the weight percentage of the coating of the sample can be calculated.

### Polymer Molecular Weights

Gel Permeation Chromatography with Multi-Angle Light Scattering Detection (GPC-MALS) may be used for determining the molecular weight of the elastic film-forming polymers herein. Molecular weights referred to herein are the weight-average molar mass (Mw). A suitable system for making these measurements consists of a DAWN DSP Laser Photometer (Wyatt Technology), an Optilab DSP Interferometric Refractometer (Wyatt Technology), and a standard HPLC pump, such as a Waters 600E system, all run via ASTRA software (Wyatt Technology).

As with any chromatographic separation, the choice of solvent, column, temperature and elution profiles and conditions depends upon the specific polymer which is to be tested. The following conditions have been found to be generally applicable for the elastic film-forming polymers referred to herein: Tetrahydrofuran (THF) is used as solvent and mobile phase; a flow rate of 1 mL/min is passed through two 300 x 7.5mm, 5µm, PLgel, Mixed-C GPC columns (Polymer Labs) which are placed in series and are heated to 40-45°C (the Optilab refractometer is held at the same temperature); 100 µL of a 0.2% polymer solution in THF solution is injected for analysis. The dn/dc values are obtained from the literature where available or calculated with ASTRA utility. The weight-average molar mass (Mw) is calculated by the ASTRA software using the Zimm fit method.

### Moisture Vapor Transmission Rate Method (MVTR method)

MVTR method measures the amount of water vapor that is transmitted through a film under specific temperature and humidity (ambient). The transmitted vapor is absorbed by CaCl₂ desiccant and determined gravimetrically. Samples are evaluated in triplicate, along with a reference film sample of established permeability (e.g., Exxon Exxaire microporous material #XBF-110W) that is used as a positive control.

This test uses a flanged cup (machined from Delrin (McMaster-Carr Catalog #8572K34) and anhydrous CaCl₂ (Wako Pure Chemical Industries, Richmond, Va.; Catalog 030-00525). The height of the cup is 55 mm with an inner diameter of 30 mm and an outer diameter of 45 mm. The cup is fitted with a silicone gasket and lid containing 3 holes for thumb screws to completely seal the cup. Desiccant particles are of a size to pass through a No. 8 sieve but not through a No. 10 sieve. Film specimens approximately 1.5" x 2.5" that are free of obvious defects are used for the analysis. The film must completely cover the cup opening, A, which is 0.0007065 m².

The cup is filled with anhydrous CaCl₂ to within 1 cm of the top. The cup is tapped on the counter 10 times, and the CaCl₂ surface is levelled. The amount of CaCl₂ is adjusted until the headspace between the film surface and the top of the CaCl₂ is 1.0 cm. The film is placed on top of the cup across the opening (30 mm) and is secured using the silicone gasket, retaining ring, and thumb screws. Properly installed, the specimen should not be wrinkled or stretched. The sample assembly is weighed with an analytical balance and recorded to ± 0.001 g. The assembly is placed in a constant temperature (40 ± 3°C) and humidity (75 ± 3% RH) chamber for 5.0 hr ± 5 min. The sample assembly is removed, covered with Saran Wrap^{®} and is secured with a rubber band. The sample is equilibrated to room temperature for 30 min, the plastic wrap removed, and the assembly is reweighed and the weight is recorded to ± 0.001 g. The absorbed moisture Mₐ is the difference in initial and final assembly weights. MVTR, in g/m²/24hr (g/m²/day), is calculated as: $MVTR = {M}_{a} / \left(A*0.208 day\right)$

Replicate results are averaged and rounded to the nearest 100 g/m²/24hr, e.g., 2865 g/m²/24hr is herein given as 2900 g/m²/24hr and 275 g/m²/24hr is given as 300 g/m²/24hr.

### Method to determine the water-swelling capacity of the film-forming polymer

The weight of the polymer specimen after soaking for 3 days in an excess of deionized water at room temperature (25 °C) is taken as W₁. The weight of this polymer specimen before drying is taken as W0. The water swelling capacity is then calculated as follows: $WSC \left[g/g\right] = \left({W}_{1}-{W}_{0}\right) / {W}_{0}$

The water swelling capacity is the water uptake of the polymer specimen in g water per 1 g of dry polymer. For this test method it is necessary to prepare polymer specimen that are typically not thicker than 1.0 mm for moderately swelling polymers. It may be necessary to prepare polymer films of less than 0.5 mm thickness for low swelling polymers in order to obtain equilibrium swelling after 3 days. A person skilled in the art will adjust the thickness and dry sample weight in a way to obtain equilibrium swelling conditions after 3 days.

### Method to determine the Theoretical Equivalent Shell/Coating Caliper of the water-absorbing material herein

If the amount of film forming polymer comprised in the water-absorbing material is known, a theoretical equivalent average caliper may be determined as defined below.

This method calculates the average caliper of a coating layer or shell on the water-absorbing material herein, under the assumption that the water-absorbing material is to be monodisperse and spherical (which may not be the case in practice). It is believed that even in the case of irregular shaped particles this method gives a good estimate for the average calliper of the shell.

### Key Parameters

| INPUT Parameter | Symbol |
|---|---|
| Mass Median Particle Size of the water-absorbing polymer (AGM) prior to coating with the film-forming polymer (also called "average diameter") | D_AGM_dry |
| Intrinsic density of the base water-absorbing polymer (bulk phase, without coating) | Rho_AGM_intrinsic |
| Intrinsic density of the film-forming elastomeric polymer (coating or shell only) | Rho_polymer shell |
| Coating (shell) Weight Fraction of the coated water-absorbing polymer (Percent of film-forming polymer coating as percent of total coated water-absorbing polymer) | c_shell_per_total |

| OUTPUT Parameters | |
|---|---|
| Average film-forming polymer coating caliper if the water-absorbing polymer is monodisperse and spherical | d_shell |
| Mass Median Particle Size of the coated water-absorbing polymer ("average diameter after coating") | D_AGM_coated |
| Coating Weight Ratio as Percent of Polymer Coating in percent of uncoated water-absorbing polymer weight | c_shell_to_bulk |

### Formulas

(note: in this notation: all c which are in percent have ranges of 0 to 1 which is equivalent to 0 to 100%.) $d_dell : = \frac{D_AGM_dry}{2} ⋅ \left[{\left[1+\frac{c_cell_per_total}{\left(1-c_cell_per_total\right)}⋅\frac{Rho_AGM_intrinsic}{Rho_polymer_shell}\right]}^{\frac{1}{3}}-1\right]$ $D_coated_AGM : = D_AGM_dry + 2 ⋅ d_shell$ $c_shell_to_bulk : = \frac{c_cell_per_total}{1 - c_cell_per_total}$

### Example calculation:

D_AGM_dry:=0.4mm (400µm); Rho_AGM_intrinsic:=Rho_polymer_shell:=1.5 g/cc

| | | | | | | | | |
|---|---|---|---|---|---|---|---|---|
| C_shell_per_total [%] | 1 | 2 | 5 | 10 | 20 | 30 | 40 | 50 |
| C_shell_to_bulk [%] | 1.0 | 2.0 | 5.3 | 11 | 25 | 43 | 67 | 100 |
| d_shell [µm] | 0.7 | 1.4 | 3.4 | 7.1 | 15 | 25 | 37 | 52 |
| D_Coated_AGM [µm] | 401 | 403 | 407 | 414 | 431 | 450 | 474 | 504 |

## Claims

1. An absorbent structure for use in an absorbent article, said absorbent structure comprising a water-absorbing material, which comprises water-absorbing polymer particles coated with a polyether polyurethane which is elastomeric, having polyalkylene oxide units in at least the side chains.

2. An absorbent structure according to claim 1 for use in an absorbent article, said absorbent structure comprising a water-absorbing material obtainable by a process comprising the steps of:
a) spray-coating water-absorbing polymer particles with said polyether polyurethane polymers that are elastomeric at temperatures in the range from 0°C to 50°C, to obtain coated particles; and
b) heat-treating the coated particles at a temperature above 50°C.

3. An absorbent structure according to claim 1 for use in an absorbent article, said absorbent structure comprising a water-absorbing material, which comprises coated water-absorbing polymer particles that have a heat- treated, spray-coating of said polyether polyurethane polymers that are elastomeric.

4. An absorbent structure according to claim 1, wherein the polyurethane is a polyetherpolyurethane that has a fraction of alkylene glycol units in the side chains which is from 10% to 90% by weight based on the total weight of the polyetherpolyurethane.

5. An absorbent structure according to claim 1, wherein the polyurethane is a polyetherpolyurethane that has ethylene oxide units in its side chain, and, optionally, in its main chains, wherein the fraction of ethylene oxide units in the side chains of the polyetherpolyurethane is not less than 12% by weight and the fraction of ethylene oxide units in the main chains of the polyetherpolyurethane is not more than 30% by weight based on the total weight of the polyetherpolyurethane.

6. An absorbent structure according to claim 1 wherein the water- absorbing material is obtainable by applying said polyether polyurethanepolymer in an amount of 0.1-25 parts by weight (based in its weight as solids material) to 100 parts by weight of dry water- absorbing polymeric particles.

7. An absorbent structure according to claim 1 wherein the water- absorbing material is obtainable by coating the water-absorbing polymeric particles by spraying with a dispersion/solution of the said polyether polyurethane polymer.

8. An absorbent structure according to claim 7 wherein said dispersion/solution is an aqueous dispersion.

9. An absorbent structure according to claim 7 wherein said dispersion/solution has a viscosity of less than 500mPas.

10. An absorbent structure according to claim 1, wherein the water- absorbing material comprises a deagglomeration aid.

11. An absorbent structure according to claim 1, wherein the water- absorbing polymers are post-crosslinked.

12. An absorbent structure according to claim 1, comprising:
a) a substrate layer, said substrate layer having a first surface and a second surface;
b) a discontinuous layer of said water-absorbing material, said discontinuous layer of water-absorbing material comprising a first surface and a second surface,
c) a layer of thermoplastic material, comprising a first surface and a second surface,
wherein the second surface of said discontinuous layer of water-absorbing material is in at least partial contact with said first surface of said substrate layer and wherein portions of said second surface of said layer of thermoplastic material are in direct contact with said first surface of said substrate layer and portions of said second surface of the said layer of thermoplastic material are in direct contact with said first surface of said discontinuous layer of water-absorbing material, wherein said thermoplastic material is preferably a hot melt adhesive, preferably a fibrous thermoplastic material.

13. An absorbent article that comprises the absorbent structure according to claim 1.

14. An absorbent article according to claim 13 wherein said absorbent article is a diaper.

15. An absorbent article according to claim 13 wherein said absorbent article is a catamenial device.

16. An absorbent article according to claim 13, wherein the absorbent structure comprises at least a part of a storage layer of the article, said absorbent structure having a density of at least about 0.4 g/cm3.

17. An absorbent article according to claim 16, wherein said storage layer comprises less than 20% by weight (of the water-absorbing material) of absorbent fibrous material.

## Patentansprüche

1. Absorptionsstruktur zum Gebrauch in einem Absorptionsartikel, wobei die Absorptionsstruktur ein wasserabsorbierendes Material umfasst, das wasserabsorbierende Polymerteilchen umfasst, die mit einem Polyetherpolyurethan beschichtet sind, das elastomer ist und Polyalkylenoxideinheiten in mindestens den Seitenketten aufweist.

2. Absorptionsstruktur nach Anspruch 1 zum Gebrauch in einem Absorptionsartikel, wobei die Absorptionsstruktur ein wasserabsorbierendes Material umfasst, das durch ein Verfahren hergestellt werden kann, das die folgenden Schritte umfasst:
a) Sprühbeschichten von wasserabsorbierenden Polymerteilchen mit den Polyetherpolyurethan-Polymeren, die bei Temperaturen im Bereich von 0 °C bis 50 °C elastomer sind, um beschichtete Teilchen herzustellen, und
b) Wärmebehandeln der beschichteten Teilchen bei einer Temperatur über 50 °C.

3. Absorptionsstruktur nach Anspruch 1 zum Gebrauch in einem Absorptionsartikel, wobei die Absorptionsstruktur ein wasserabsorbierendes Material umfasst, das beschichtete wasserabsorbierende Polymerteilchen umfasst, die eine wärmebehandelte Sprühbeschichtung der Polyetherpolyurethan-Polymere aufweisen, die elastomer sind.

4. Absorptionsstruktur nach Anspruch 1, wobei das Polyurethan ein Polyetherpolyurethan ist, das eine Fraktion von Alkylenglycoleinheiten in den Seitenketten aufweist, die 10 Gew.-% bis 90 Gew.-% bezogen auf das Gesamtgewicht des Polyetherpolyurethans ausmacht.

5. Absorptionsstruktur nach Anspruch 1, wobei das Polyurethan ein Polyetherpolyurethan ist, das Ethylenoxideinheiten in seiner Seitenkette und wahlweise in seinen Hauptketten aufweist, wobei die Fraktion von Ethylenoxideinheiten in den Seitenketten des Polyetherpolyurethans nicht weniger als 12 Gew.-% ausmacht und die Fraktion von Ethylenoxideinheiten in den Hauptketten des Polyetherpolyurethans nicht mehr als 30 Gew.-% bezogen auf das Gesamtgewicht des Polyetherpolyurethans ausmacht.

6. Absorptionsstruktur nach Anspruch 1, wobei das wasserabsorbierende Material durch Auftragen des Polyetherpolyurethan-Polymers in einer Menge von 0,1-25 Gewichtsteilen (bezogen auf sein Gewicht als Feststoffmaterial) bis 100 Gewichtsteilen trockener wasserabsorbierender Polymerteilchen herstellbar ist.

7. Absorptionsstruktur nach Anspruch 1, wobei das wasserabsorbierende Material durch Beschichtung der wasserabsorbierenden Polymerteilchen durch Besprühen mit einer Dispersion/Lösung des Polyetherpolyurethan-Polymers herstellbar ist.

8. Absorptionsstruktur nach Anspruch 7, wobei die Dispersion/Lösung eine wässrige Dispersion ist.

9. Absorptionsstruktur nach Anspruch 7, wobei die Dispersion/Lösung eine Viskosität von weniger als 500 mPas aufweist.

10. Absorptionsstruktur nach Anspruch 1, wobei das wasserabsorbierende Material ein Deagglomerationshilfsmittel umfasst.

11. Absorptionsstruktur nach Anspruch 1, wobei die wasserabsorbierenden Polymere nachträglich vernetzt werden.

12. Absorptionsstruktur nach Anspruch 1, umfassend:
a) eine Substratschicht, wobei die Substratschicht eine erste Oberfläche und eine zweite Oberfläche aufweist,
b) eine diskontinuierliche Schicht des wasserabsorbierenden Materials, wobei die diskontinuierliche Schicht von wasserabsorbierendem Material eine erste Oberfläche und eine zweite Oberfläche umfasst,
c) eine Schicht aus thermoplastischem Material, die eine erste Oberfläche und eine zweite Oberfläche umfasst,
wobei die zweite Oberfläche der diskontinuierlichen Schicht von wasserabsorbierendem Material mindestens teilweise in Kontakt mit der ersten Oberfläche der Substratschicht ist und wobei Abschnitte der zweiten Oberfläche der Schicht aus thermoplastischem Material in direktem Kontakt mit der ersten Oberfläche der Substratschicht ist und Abschnitte der zweiten Oberfläche der Schicht aus thermoplastischem Material in direktem Kontakt mit der ersten Oberfläche der diskontinuierlichen Schicht von wasserabsorbierendem Material ist, wobei das thermoplastische Material vorzugsweise ein Schmelzkleber, vorzugsweise ein thermoplastisches Fasermaterial ist.

13. Absorptionsartikel, der die Absorptionsstruktur nach Anspruch 1 umfasst.

14. Absorptionsartikel nach Anspruch 13, wobei der Absorptionsartikel eine Windel ist.

15. Absorptionsartikel nach Anspruch 13, wobei der Absorptionsartikel eine Menstruationsvorrichtung ist.

16. Absorptionsartikel nach Anspruch 13, wobei die Absorptionsstruktur mindestens einen Teil einer Speicherschicht des Artikels umfasst, wobei die Absorptionsstruktur eine Dichte von mindestens ungefähr 0,4 g/cm3 aufweist.

17. Absorptionsartikel nach Anspruch 16, wobei die Speicherschicht zu weniger als 20 Gew.-% (des wasserabsorbierenden Materials) absorbierendes Fasermaterial umfasst.

## Revendications

1. Structure absorbante utilisable dans un article absorbant, ladite structure absorbante comprenant un matériau absorbant l'eau, qui comprend des particules polymères absorbant l'eau revêtues d'un polyéther polyuréthane qui est élastomère, ayant des motifs poly(oxyde d'alkylène) au moins dans les chaînes latérales.

2. Structure absorbante selon la revendication 1 utilisable dans un article absorbant, ladite structure absorbante comprenant un matériau absorbant l'eau pouvant être obtenu par un procédé comprenant les étapes consistant à :
a) revêtir par pulvérisation des particules polymères absorbant l'eau avec lesdits polymères de polyéther polyuréthane qui sont élastomères à des températures dans la gamme de 0 °C à 50 °C, de façon à obtenir des particules revêtues ; et
b) traiter thermiquement les particules revêtues à une température supérieure à 50 °C.

3. Structure absorbante selon la revendication 1 utilisable dans un article absorbant, ladite structure absorbante comprenant un matériau absorbant l'eau, qui comprend des particules polymères absorbant l'eau revêtues qui ont un revêtement par pulvérisation traité thermiquement desdits polymères de polyéther polyuréthane qui sont élastomères.

4. Structure absorbante selon la revendication 1, dans laquelle le polyuréthane est un polyéther-polyuréthane qui a une fraction de motifs alkylène glycol dans les chaînes latérales qui va de 10 % à 90 % en poids sur base du poids total du polyéther-polyuréthane.

5. Structure absorbante selon la revendication 1, dans laquelle le polyuréthane est un polyéther-polyuréthane qui a des motifs oxyde d'éthylène dans sa chaîne latérale, et, facultativement, dans ses chaînes principales, dans laquelle la fraction de motifs oxyde d'éthylène dans les chaînes latérales du polyéther-polyuréthane n'est pas inférieure à 12 % en poids et la fraction de motifs oxyde d'éthylène dans les chaînes principales du polyéther-polyuréthane n'est pas supérieure à 30 % en poids sur base du poids total du polyéther-polyuréthane.

6. Structure absorbante selon la revendication 1, dans laquelle le matériau absorbant l'eau peut être obtenu en appliquant le polymère élastomère en une quantité de 0,1 à 25 parties en poids (sur base de son poids en tant que matériau solide) à 100 parties en poids de particules polymères absorbant l'eau sèches.

7. Structure absorbante selon la revendication 1, dans laquelle le matériau absorbant l'eau peut être obtenu par revêtement des particules polymères absorbant l'eau par pulvérisation avec une dispersion/solution dudit polymère polyéther polyuréthane.

8. Structure absorbante selon la revendication 7, dans laquelle ladite dispersion/solution est une dispersion aqueuse.

9. Structure absorbante selon la revendication 7, dans laquelle ladite dispersion/solution a une viscosité inférieure à 500 mPas.

10. Structure absorbante selon la revendication 1, dans laquelle le matériau absorbant l'eau comprend un auxiliaire de désagglomération.

11. Structure absorbante selon la revendication 1, dans laquelle les polymères absorbant l'eau sont post-réticulés.

12. Structure absorbante selon la revendication 1, comprenant :
a) une couche de substrat, ladite couche de substrat ayant une première surface et une deuxième surface ;
b) une couche discontinue dudit matériau absorbant l'eau, ladite couche discontinue du matériau absorbant l'eau comprenant une première surface et une deuxième surface,
c) une couche de matériau thermoplastique, comprenant une première surface et une deuxième surface,
dans laquelle la deuxième surface de ladite couche discontinue de matériau absorbant l'eau est en contact au moins partiel avec ladite première surface de ladite couche de substrat et dans laquelle des parties de ladite deuxième surface de ladite couche de matériau thermoplastique sont en contact direct avec ladite première surface de ladite couche de substrat et des parties de ladite deuxième surface de ladite couche de matériau thermoplastique sont en contact direct avec ladite première surface de ladite couche discontinue de matériau absorbant l'eau, dans laquelle ledit matériau thermoplastique est de préférence un adhésif thermofusible, de préférence un matériau thermoplastique fibreux.

13. Article absorbant qui comprend la structure absorbante selon la revendication 1.

14. Article absorbant selon la revendication 13, où ledit article absorbant est une couche.

15. Article absorbant selon la revendication 13, où ledit article absorbant est un dispositif cataménial.

16. Article absorbant selon la revendication 13, dans lequel la structure absorbante comprend au moins une partie d'une couche de stockage de l'article, ladite structure absorbante ayant une masse volumique d'au moins environ 0,4 g/cm³.

17. Article absorbant selon la revendication 16, dans lequel ladite couche de stockage comprend moins de 20 % en poids (du matériau absorbant l'eau) de matériau fibreux absorbant.
